# EUROPEAN PATENT APPLICATION

(11) **EP 1 820 538 A1**
(43) Date of publication of application: **22.08.2007**
(21) Application number: 05781374.3
(22) Date of filing: 02.09.2005
(51) Int. Cl.: A61Q 19/00, A61K 8/73

(54) **COSMETICS AND PROCESS FOR PRODUCTION THEREOF**

(30) Priority: 07.09.2004 JP 2004259433
(71) Applicant: Hakuto Co., Ltd, Shinjuku-ku, Tokyo 160-8910 (JP)
(72) Inventor: NOHATA, Yasuhiro Yokkaichi Lab., HAKUTO CO. LTD., Yokkaichi-shi, Mie 5100007 (JP); TAJIMA, Kazuo, Yokohama-shi, Kanagawa 233-0001 (JP); IMAI, Yoko, Tokyo 158-0091 (JP); HORIUCHI, Teruo, Saitama 350-1308 (JP)
(74) Representative: Pautex Schneider, Nicole Véronique
(86) International application number: PCT/JP2005/016108
(87) International publication number: WO 2006/028012

(57) **Abstract**

An emulsion and dispersion system exhibiting excellent stability against heat and excellent stability over a long period time in interfaces between functional oily bases and water, or between functional granules as solid particles and water, etc., a cosmetic composition containing an emulsifying dispersant which is capable of performing emulsification and dispersion independently of required HLB values of the functional oily bases or the surface states of the functional granules, and a producing method of such a cosmetic composition. A cosmetic composition **characterized in that** an emulsifying dispersant including polysaccharides with particulate structure as a main component, and components to be emulsified are contained, and a producing method thereof. And a cosmetic composition **characterized in that** the emulsifying dispersant exists as a three-phase emulsifying dispersant which adheres to a periphery of the components to be emulsified like a layer to form an intermediate layer, and a producing method thereof.

## Description

### Technical Field of the Invention

The present invention relates to a cosmetic composition which contains an emulsifying dispersant exhibiting excellent stability over a long period of time, irrespective of kinds of components to be emulsified, and a method for producing the same.

### Prior Art

The cosmetic composition contains various many components according to the kind thereof, and, for example, contains organic solvents, organic coloring agents, inorganic coloring agents, Oils, moisture retaining agents, astringents, bleaching agents, UV preventive agents, oxidation preventive agents and perfumes as functional oily bases or functional granules. The functional oily bases or functional granules have the cleaning effect, water-retaining effect, scrubbing effect, emolient effect and protection effect against skins, and perform the functions of improving the spreading ability, smooth feeling, glossiness, adhering properties, etc. of the cosmetic composition.

Where the functional oily bases or functional granules are used in the cosmetic composition, these components are emulsified or dispersed in purified water, using surfactants.

In the case of the functional oily bases, the surfactants have been selected according to required HLB values thereof and characteristics of surfaces of granules, and the emulsion and dispersion have been performed using many kinds of surfactants. The required HLB values of the surfactants adapted to be used as the emulsifying dispersants must be made different between the case of oil-in water(O/W) emulsions being produced and the case of water-in-oil (W/O) emulsions being produced, and the sufficient stability against heat and the sufficient stability over a long period of time are not exhibited so that many and various kinds of surfactants have been used as mixtures thereof (see "Emulsion Science" Edited by P. Sherman, Academic PressInc. (1969), "Microemulsions-Theory and Practice-Edited by Leon M. price, Academic Press Inc. (1977), "Technique of Emulsification and Solubilization" edited by Susumu Tsuji, Kougakutosho Ltd. (1976), "Developing Technique of Functional Surfactants" published by CMC Publishing Co., Ltd. (1998), etc.

### Disclosure of the Invention

### Problems to be Solved by the Invention

In the conventional emulsifying method using surfactants, the basic emulsifying method is to adsorb surfactants on interfaces between oil and water to decrease surface energy therein so that a large amount of emulsifiers have been needed to reduce interfacial tension thereof.

The surfactants, however, are low in biodegradation to cause bubbling, and accordingly, exhibit the problems such as environmental pollution so that the reduction of the amount of the surfactants have been required.

And emulsifiers for functional oil bases have been generally prepared by physicochemical emulsifying methods such as the HLB method, phase transition emulsifying method, phase transition temperature emulsifying method, gel emulsifying method, etc, and in these methods, the emulsions have been prepared by decreasing surface energies in oil/water interfaces to stabilize systems thermodynamically. Therefore, in order to select the emulsifiers mostly suited to the cosmetic compositions, very troublesome and lots works have been needed, and since in the cosmetic compositions, many kinds of oily bases are mixed therein, stable emulsification thereof has been frequently difficult.

In addition, the cosmetic compositions are required to exhibit many and various effects so that it is needed to stabilize many substances exhibiting various interfacial tensions in a single cosmetic composition. In particular, silicone oil is chemically stable, safe with substantially no reactivity, odorless with little stickiness, have a low surface tension with good spreading ability, and have smooth feeling so that it has been blended in many cosmetic compositions such as creams, milky lotions, lotions, jells, etc.

However, since silicone oil is very hydrophobic and the interfacial tension thereof is low, it has been difficult to stabilize in aqueous solutions. And where the surfactants adapted to emulsify silicone oil are used, there has occurred the problem that emulsification of organic acids such as stearic acid, etc. and higher alcohols such as cetanol, etc. become instable, and in the case of the substances such as titanium oxide particles and iron oxide particles, of which interfacial tensions are extremely different from that of organic chemical compounds, there has occurred the problem that emulsification becomes more difficult.

And when a large amount of oily bases are blended in water, there occur phase transitions in "type" of emulsion, and where surfactants are used, oily bases phases and water phases may separate from each other.

In contrast, the present inventors have studied intensively to develop cosmetic compositions prepared using a novel emulsifying technique in the emulsifying method of oily bases. As a result, they have developed the cosmetic compositions prepared using the three-phase emulsifying method of carrying out emulsification by making nanoparticles which exist in systems of oil/amphipathic chemical compounds/water as independent phases adhere to surfaces of oily bases with van der Waals' force. And with such emulsifying method, it has been noted that not required HLB values of oily components but the interfacial tensions of oily components/water interfaces are important for the adhesion of the nanoparticles of emulsifiers. In addition, the present inventors have found that this three-phase emulsion exhibits very high stability, as compared with conventional O/W type emulsions prepared with normally used surfactants.

And, with respect to the dispersing method of solid particles contained in cosmetic compositions, the conventional dispersing method using the surfactants has basically adopted the dispersing method of making the surfactants adsorb on surfaces of solid bodies to prevent flocculation of the solid bodies with the protective colloid effect of adsorption layers. With this method, in order to form the adsorption layers, a large amount of surfactants has been needed, similarly the case of the oily bases. In addition, in order to keep the amount of the surfactants on the surfaces of the solid bodies, the concentration of the surfactants in solvents has been needed to be elevated to increase the equilibrium pressure.

In order to overcome the above-described problems, the present inventors have found the three-phase emulsifying and dispersing method of performing emulsification and dispersion by making nanoparticles which exist in a system of solid substances/amphipathic chemical compounds/water as independent phases, similarly to the oily bases, adhere to the surfaces of the solid particles with van der Waals' force. And with such emulsifying and dispersing method, it has been noted that not surface tensions of the solid particles but interfacial tensions in interfaces between solid particles components/water are important for the adhesion of the nanoparticles of dispersants. In addition, the present inventors have found that this three-phase emulsifying dispersants exhibit a very high stability as compared with the the dispersion and blocking of flocculation of the solid particles with normally used surfactants, and have completed the present invention based on these findings.

Under the above-described circumstances, the object of the present invention is to form an emulsion and dispersion system excellent in stability against heat and stability over a long period of time in interfaces between functional oily bases and water, or between functional granules as solid particles and water, etc., which are to be used in the cosmetic composition, and to provide cosmetic compositions containing emulsifying dispersants which are capable of performing emulsification and dispersion independently of required HLB values of the functional oily bases or surface states of the functional granules, and the producing method of such cosmetic compositions.

### Means for Solving the Problems

The present invention according to claim 1 provides a cosmetic composition characterized in that the cosmetic composition contains an emulsifying dispersant which includes polysaccharides with particulate structures as a main component, and further contains components to be emulsified.

Though the conventional surfactants have required a large amount of emulsifiers, in accordance with the present invention, since emulsifying dispersants including polysaccharides with particulate structures as a main component and components to be emulsified are contained, very stable emulsified cosmetic compositions can be obtained with the three-phase emulsification of making nanoparticles of polysaccharides with particulate structures, which exist in a system of oil/amphipathic chemical compounds/water as independent phases, adhere to surfaces of oily bases with van der Waals' force. And with the dispersing method of the solid particles, flocculation thereof can be prevented, as compared with the conventional dispersing method using surfactants.

The invention according to claim 2 is a cosmetic composition characterized in that the emulsifying dispersant containing polysaccharides with particulate structures as a main component exists as a three-phase emulsifying dispersant which adheres to a periphery of the component to be emulsified like a layer to define an intermediate layer.

The polysaccharides with particulate structures can adhere to the periphery of the component to be emulsified with van der Waals' force like a layer to form the intermediate layer irrespective of the properties of the components to be emulsified so that the components to be emulsified can be stably emulsified and the emulsion state can be maintained. Namely, emulsifying dispersant phases are formed in interfaces between the components to be emulsified, dispersing materials and solvents so that the components to be emulsified and dispersed are difficult to incorporate with each other after emulsification, and accordingly, resultant emulsion and dispersion layers are very excellent in stability against heat and stability over a long period of time irrespective of the kinds of the components to be emulsified.

The invention according to claim 3 is a cosmetic composition in which the polysaccharides with particulate structures have an average particle diameter ranging from 8 nm to 500 nm.

When the average particle diameter is in the range from 8 nm to 500 nm, there can be obtained the emulsion and dispersion state wherein the components to be emulsified and dispersed become stable. When the average particle diameter is less than 8 nm, the attracting function due to the van der Waals' force is decreased, and consequently, vesicle particles become difficult to adhere to surfaces of oil drops, and when the average particle diameter is greater than 500 nm, stable eumusions cannot be maintained.

The invention according to claim 4 is a cosmetic composition wherein the polysaccharides with particulate structures are polysaccharides which are formed into compact globules.

Since the polysaccharides are formed into compact globules, a large number of compact globules adhere to a periphery of the component to be emulsified like a layer to form an intermediate layer, and the emulsifying dispersant formed into compact globules stably exists as a third phase so that the emulsion state can be maintained over a long period of time.

The invention according to claim 5 is a cosmetic composition wherein the polysaccharides with particulate structures contain at least one of fucose, glucose, glucuronic acid and rhamnose as a constituting monosaccharide, and further contain fucose and/or rhamnose in side chains thereof.

Since these polysaccharides contain the above-described constituting monosaccharide, and further contains fucose and/or rhamnose in the side chains thereof, the polysaccharides with particulate structures retain the components to be emulsified inside thereof to form a construction of which an outer surface forms a phase exhibiting hydrophilic properties and lyophilic properties, thereby forming a three-phase structure composed of components to be emulsified and dispersed- polysaccharides with particulate structures-solvents.

The invention according to claim 6 is a cosmetic composition wherein the polysaccharides with particulate structures include at least the polysaccharide represented by the following formula (1).

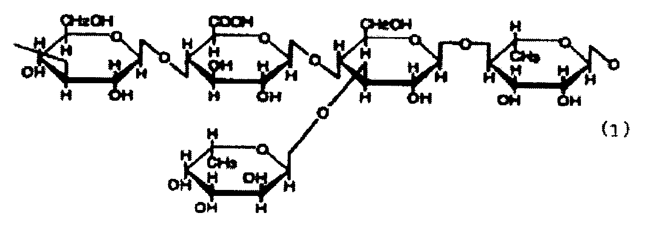

Since the polysaccharide represented by the formula (1) is contained, the particulate structure can be formed, and since the outer surface thereof contains hydrophilic groups such as OH groups, the polysaccharides with particulate structures retain components to be emulsified inside thereof, and the outer surface exhibits hydrophilic properties and lyophilic properties, thereby forming a three-phase structure composed of components to be emulsified and dispersed- polysaccharides with particulate structures-solvents.

The invention according to claim 7 is a cosmetic composition wherein the emulsifying dispersant containing polysaccharides with particulate structures as a main component exists in the weight ratio of from 1 : 50 to 1 : 1000 to the components to be emulsified.

By virtue of the particulate structures, even a small amount of emulsifying dispersant relative to the components to be emulsified and dispersed can serve to obtain stable emulsified and dispersed substances.

Where the weight ratio of the emulsifying dispersants to the components to be emulsified is less than the ratio of 1 : 50, the amount of the emulsifying dispersants increases to raise the production costs, and where the weight ratio is greater than the ratio of 1 : 1000, it becomes difficult to form such particulate structures. For these reasons, the preferable range of the weight ratio is from 50 to 1000.

The invention according to claim 8 is a cosmetic composition wherein the emulsifying dispersant which includes the polysaccharides with particulate structures as a main component contains urea.

Since the emulsifying dispersant includes urea, the emulsifying dispersant including the polysaccharides with particulate structures can be readily formed into compact globules, and the components to be emulsified and dispersed are wrapped therewith to form a stable three-phase construction. It is preferable that the content of urea ranges from 0.1 to 10 wt% relative to the entire amount of the cosmetic composition.

The invention according to claim 9 is a cosmetic composition wherein the components to be emulsified are functional oily bases. By virtue of the polysaccharides with particulate structures, many kinds of functional oily bases which are contained in the cosmetic composition with a large amount can maintain the emulsion state stably over a long period of time.

The invention according to claim 10 is a cosmetic composition wherein one of the components to be emulsified is silicone oil. By virtue of the polysaccharides with particulate structures in accordance with the present invention, the emulsion state of silicone oil which is contained in the cosmetic composition with a large amount but is difficult to maintain the emulsion state can be maintained stably over a long period of time, which is preferable as the cosmetic composition.

The invention according to claim 11 is a cosmetic composition wherein one of the components to be emulsified is titanium oxide particles and/or titanium oxide particles subjected to surface treatments. By virtue of the polysaccharides with particulate structures in accordance with the present invention, the dispersion state of titanium oxide particles and/or titanium oxide particles subjected to surface treatments which are contained in the cosmetic composition with a large amount but are difficult to maintain the dispersion state can be maintained stably over a long period of time.

The invention according to claim 12 is a method for producing a cosmetic composition including the steps of bringing an emulsifying dispersant containing polysaccharides with particulate structures as a main component thereof into contact with components to be emulsified and/or components to be dispersed, mixing them to emulsify and/or dispersing them, and blending other components for the cosmetic composition therein.

Though conventional surfactants have required a large amount of emulsifiers, with the present invention, since the components to be emulsified are emulsified using the emulsifying dispersant which contains the polysaccharides with particulate structures as a main component, the three-phase emulsification is performed by making nanoparticles which exist in a system of oil/ amphipathic chemical compound/water as independent phases adhere to surfaces of oily bases with van der Waals' force, and other components for the cosmetic composition are blended to adjust the cosmetic composition. With a small amount of emulsifying dispersant, very stable emulsified cosmetic composition can be produced.

Furthermore, as compared with the conventional dispersing method of solid particles using surfactants, the flocculation of the solid bodies can be prevented.

The invention according to claim 13 is a method for producing a cosmetic composition wherein the emulsifying dispersant which includes polysaccharides with particulate structures as a main component is mixed with the components to be emulsified and/or the components to be dispersed in the weight ratio of from 1 : 50 to 1 : 1000.

With this producing method, even a small amount of emulsifying dispersant can serve to obtain stable cosmetic composition.

Where the weight ratio of the emulsifying dispersant to the components to be emulsified is less than the ratio of 1 : 50, the amount of the emulsifying dispersant increases to raise the production costs, and where the weight ratio is greater than the ratio of 1 : 1000, it becomes difficult to form such particulate structures. For these reasons, the preferable range of the weight ratio is from 1: 50 to 1 : 1000.

The invention according to claim 14 is a producing method of a cosmetic composition wherein the polysaccharides with particulate structures contain at least one of fucose, glucose, glucuronic acid and rhamnose as a constituting monosaccharide. Since these polysaccharides are contained as the constituting monosaccharide, the polysaccharides with particulate structures retain the components to be emulsified inside thereof to form a construction of which an outer surface exhibits hydrophilic properties and lyophilic propeties, whereby the cosmetic composition with a three-phase structure composed of components to be emulsified and dispersed- polysaccharides with particulate structures-solvents can be produced.

The invention according to claim 15 is a producing method of a cosmetic composition which includes as the polysaccharides with particulate structures at least the polysaccharide represented by the following formula (1).

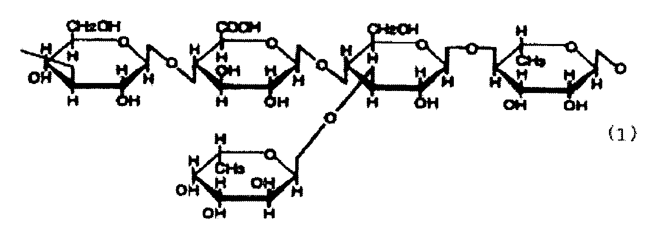

Since the polysaccharides containing the polysaccharide represented by the formula (1) are used, the polysaccharides with the particulate structures contain hydrophilic groups such as OH groups in outer surfaces thereof, and retain components to be emulsified inside thereof, whereby a three-phase structure composed of hydrophilic properties, polysaccharides with particulate structures, lyophilic properties is formed to emulsify the components to be emulsified. Thus, the cosmetic composition can be produced.

The invention according to claim 16 is a producing method of a cosmetic composition wherein the emulsifying dispersant which includes the polysaccharides with particulate structures is produced by adding urea to an aqueous solution containing the polysaccharide represented by the formula (1). Since urea is added to the aqueous solution of the emulsifying dispersant, the emulsifying dispersant containg the polysaccharides can be readily formed into compact globules to form a three-phase construction, and the components to be emulsified can be emulsified and stabilized. It is preferable that the content of urea ranges from 0.1 wt% to 10 wt% relative to the entire amount of the cosmetic composition.

### Effects of the Invention

As described above, with the present invention, the cosmetic composition contains an emulsifying dispersant which forms an emulsion and dispersion system excellent in stability against heat and stability over a long period of time in interfaces between functional oily bases and water, or between functional granules and water, etc. Therefore, though the conventional hydrocarbon-based surfactants are difficult to form stable emulsions, with the cosmetic composition of the present invention, it has become possible to obtain the cosmetic composition which exhibits stability in emulsifying and dispersing properties over a long period of time and in a wide temperature range.

In addition, it has become possible to emulsify and disperse oily components and solid particles independently of required HLB values of the oily bases to be emulsified, or the surface states of the functional granules, many kinds of which are contained in the cosmetic composition, so that the emulsification of hydrocarbon-based oils and silicone-based oils also becomes possible. Consequently, troubles and works encountered upon selecting the emulsifying dispersant suited to many kinds of components to be emulsified in the production of the cosmetic composition can be minimized, and it has become possible to emulsify many kinds of oily components and solid particles which exist as a mixture simultaneously. In addition, an emulsion system and a dispersion system can readily coexist.

Furthermore, the concentration of the emulsifying dispersant, which is required to perform the emulsification and dispersion, is as small as from 1/50 to 1/1000 of that of the conventionally used surfactants, and consequently, load on the environment can be remarkably reduced.

### The best embodiment for effecting the invention

Hereinafter, the best embodiment of the present invention will be explained while comparing with the conventional cosmetic composition.

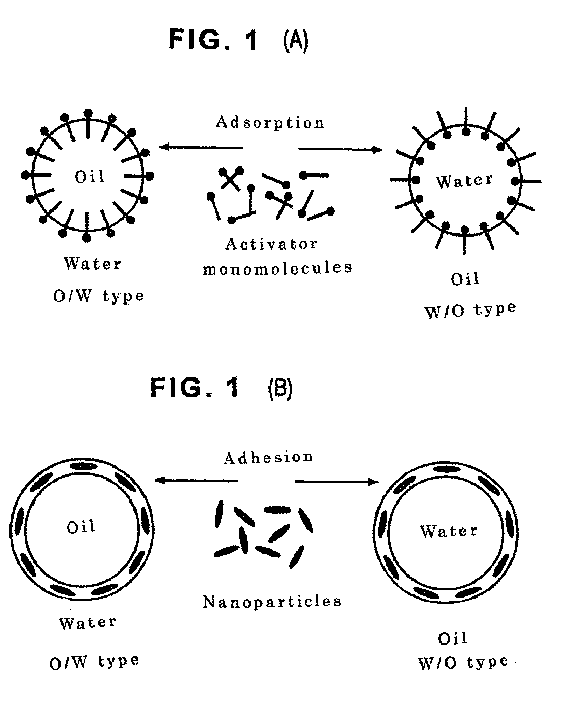

FIG. 1 shows a schematic diagram of the conventional emulsifying method using surfactants to be contained in the cosmetic composition and the three-phase emulsifying method using emulsifying dispersants to be contained in the cosmetic composition of the present invention.

In the conventional emulsifying method using surfactants, as shown in FIG. 1(A), the surfactant has hydrophilic groups and lipophilic groups which respectively have properties different from each other in each monomolecule. The lipophilic groups of the surfactant exhibit compatibility with oil, and the hydrophilic groups are arranged so as to be oriented outside the oil particles so as to become readily wet with water, and be mixed in a water medium homogeneously, thereby forming O/W type emulsions.

In addition, against water particles, the hydrophilic groups of the surfactant are oriented, whereas the lipophilic groups of the surfactant are arranged outwardly of the water particles, become oily and are mixed in an oil medium homogeneously, thereby forming W/O type emulsions.

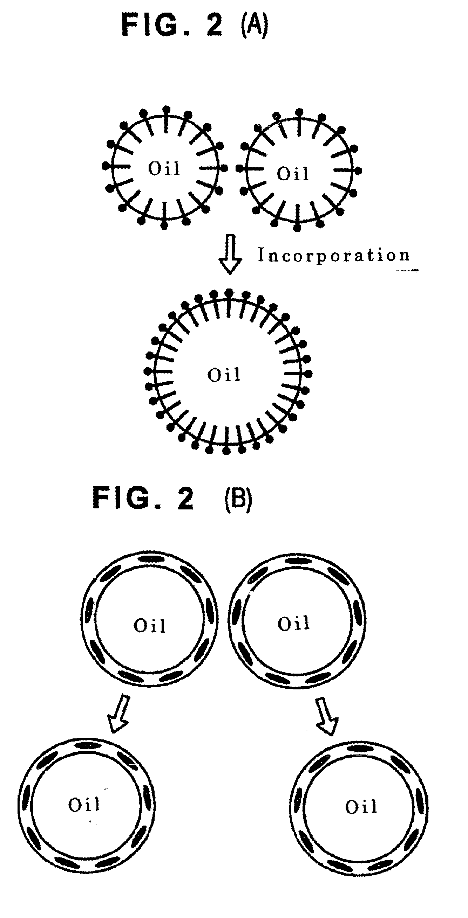

However, with the conventional emulsifying method, the surfactants are adsorbed on oil surfaces to form emulsion films like monomolecules films so that there occurs the trouble of physical properties varying according to the kinds of the surfactants. In addition, as shown in FIG. 2(a), oil drops incorporate with each other due to thermal collision thereof, and consequently, the dimensions of the oil drops gradually increase and at last, there occurs the separation into oil and an aqueous solution of surfactants. In order to prevent such separation, microemulsions must be formed, but there occurs the trouble that a large amount of surfactants must be used.

Since the lipophilic components of the surfactants have organic properties and inorganic properties due to their chemical structures, conventionally, the suitable surfactant has been selected based on indexes such as the HLB value, etc. for emulsification. However, since the cosmetics contain many lipophilic components, it is very difficult to select one surfactant or a combination of a plurality of surfactants, which are suited to all of the lipophilic components, and to this effect, many experiences must be needed. In addition, the amount of the surfactants to be used normally ranges from 5 to 20 %. This high content causes problems as the cosmetic compositions.

Under the above circumstances, in accordance with the present invention, as shown in FIG. 1 (B), by making nanoparticles in an emulsifier phase adhere to oil particles and water particles in the cosmetic composition, a three phase structure composed of water phase-emulsifying dispersant phase-oil phase is formed, and surface energy is prevented from decreasing due to the compatibility, as is different from the conventional surfactants, and, as shown in FIG. 2(B), incorporation caused by thermal collision is difficult to occur, whereby emulsions can be stabilized over a long period of time. And based on these mechanisms, a novel emulsifying method (three-phase emulsifying method) which is capable of forming emulsions with a small amount of emulsifying dispersants has been adopted.

As a result, emulsification becomes possible with a small amount of emulsifying dispersants in accordance with the present invention irrespective of the lipophilic components to be blended, and emulsions can be obtained using no surfactant or using an extremely small amount of surfactants as supplementary components.

It has been considered to use emulsifying dispersants containing polysaccharides with particulate structures as a main component as the emulsifying dispersants adapted to effect the above-described three phases emulsificaton.

The preferred average particle diameter of polysaccharides with particulate structures ranges from 8 nm to 500 nm. Where the particle diameter is less than 8 nm, the attraction operation caused by van der Waals' force decreases so that the polysaccharides become difficult to adhere to surfaces of oil drops, and where the particle diameter is greater than 500 nm, stable emulsions cannot be maintained.

The polysaccharide with a particulate structure, which is to be used in the present invention, is the polysaccharide composed of at least fucose, glucose, glucuronic acid and rhamnose as constituting monosaccharides, and, as represented by the following formula (1), preferably has main chains with a repetition construction composed of glucose, glucuronic acid and rhamnose where one fucose diverges from one glucose in the main chain.

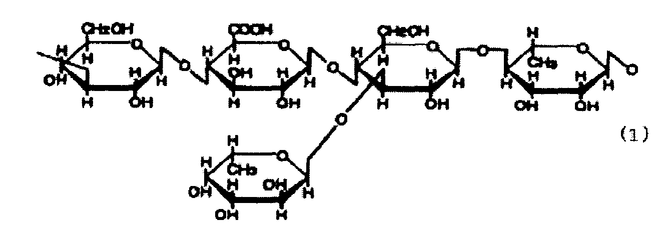

The polysaccharide represented by the formula (1) can be obtained as a product of a microorganism of Alkaligenes latus strain B-16 (FERM BP-2015), for example. The microorganism of Alkaligenes latus strain B-16 is cultured by a normal microbes culturing method, and, after cultured, organic solvents such as acetone, ethanol and isopropyl alcohol are added to a culture liquid. As a result, polysaccharide precipitates as an insoluble substance. The precipitation of polysaccharide is separated, thereby obtaining a polysaccharide.

Generally, microorganisms produce two or more kinds of polysaccharides. Other kinds of polysaccharide than the polysaccharide of the present invention may be included provided that they do not obstruct the effects of the present invention. For example, it has been proved that the polysaccharides produced by a microorganism Alkaligenes latus strain B-16 include at least two kinds of polysaccharides, and the moler ratio of the constituting monosaccharides of polysaccharides which are separated from culture liquid is:
fucose : glucose : glucuronic acid : rhamnose = 1 : (0.5 to 4) : (0.5 to 2) : (0.5 to 2)

When these two kinds of polysaccharides are separated from each other, one kind of polysaccharide is a polysaccharide having a construction wherein one fucose diverges from one glucose in the main chains with a repeated construction, which is composed of glucose, glucuronic acid and rhamnose, as shown in the formula (1), and another kind of polysaccharide is a polysaccharide of which repetition unit is composed of fucose and mannose. The former is the polysaccharide of the present invention, has a constituting ratio of fucose, glucose, glucuronic acid and rhamnose which is 1 : 2 :1 : 1, and is a high molecular component having a molecular weight of about 10⁹ [see the Japan Agricultural Chemistry Society, 1998th year Large Meeting, Summary, P. 371]. The latter is the polysaccharide having a repetition construction of fucose and mannose of 1 : 1, and is a low molecular component having a molecular weight of 10³ to 10⁷ [see Y. Nohata, J. Azuma, R. Kurane, Carbohydrate Research 293, (1996) 213 to 222]. This low molecular component is not within the scope of the polysaccharide of the present invention, but does not obstruct the stabilizing effect of the present invention so that it may be contained in the cosmetic composition. These polysaccharides have been sold on market as Alcasealan [trade name, INCI name: Alcaligenes Polysacchaides, manufactured by HAKUTO CO., LTD.].

These poysaccharides contain fucose having hydrophobic metyl groups, and fucose diverges from sugar chains so as to project outwardly thereof due to the hydrophobic methyl groups. Therefore, where the polysaccharides are formed into compact globules in a water system, relatively many fucose gather in the interiors of the globules, and consequently, the interiors thereof become under hydrophobic environments. On the other hand, hydrophilic groups such as carboxyl groups, hydroxy groups, etc. of the sugar chains of the polysaccharides are oriented outwardly, and relatively many hydrophilic groups such as carboxyl groups, hydroxy groups, etc. as polar groups are directed toward outer surfaces of the compact globules. As a result, the outer surfaces of the compact globules form hydrophilic and lyophilic structures, whereby a cosmetic composition with a three phase structure composed of components to be emulsified and dispersed-polysaccharides with particulate structures-solvents is obtained.

Where the components to be emulsified and dispersed are emulsified and dispersed using emulsifying dispersants containing polysaccharides formed into compact globules as a main component, the components to be emulsified and dispersed and the emulsifying dispersant may be made to contact each other and blend with each other in the weight ratio of from 1:50 to 1:1000. In the case of extremely many components being contained, like the case of the cosmetic composition, it is more efficient to perform emulsification and dispersion in two steps.

First, an aqueous solution of polysaccharides with a predetermined concentration in accordance with the present invention is prepared. It is efficient to use a melting device such as a homogenizer and a disperser which can apply a strong shear. The concentration of the polysaccharides ranges from 0.001 % to 1 %, preferably ranges from 0.01 % to 0.5 %, and more preferably ranges from 0.05 % to 0.2 %. In the case of 0.001 % or less, there may be the case expected effects cannot be achieved sufficiently, and in the case of 1 % or more, expected effects are obtained, but the production costs are not desirable.

Where the polysaccharides are formed to have particulate structures, and in particular form particles as compact globules, the formation of the particulate structures is accelerated by adding urea to the solution of the polysaccharides. It is preferable that the content of urea ranges from 0.1 to 10 wt% to the entire weight of the cosmetic composition.

Examples of the components to be emulsified and dispersed, which are to be used in the present invention, include silicone oil hydrocarbons, waxes, higher alcohols, fatty acid esters, organic esters, glycerides, fluoridaion hydrocarbons, etc. As described above, in many cases, silicone oil is used in the cosmetic composition.

Silicone oil to be used in the present invention is a polymer having a siloxane bond represented by the general formula (2) as a main chain thereof.

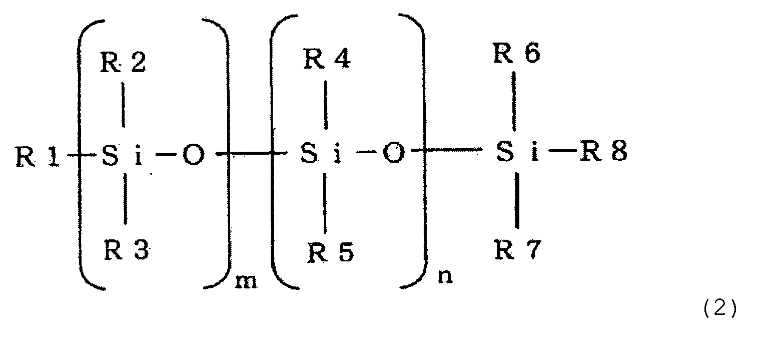

In the general formula, R1, R2, R3, R6, R7 and R8 respectively represent a hydrogen atom, a hydroxyl group, an alkyl group having 1 to 22 carbon atoms that may involve a fluorine group, an alcohol residue having 1 to 22 carbon atoms, a carboxylic acid residue having 1 to 22 carbon atoms or an aryl group having 6 to 24 carbon atoms, and R4 and R 5 respectively represent a hydrogen atom, a hydroxyl group, an alkyl group having 1 to 22 carbon atoms that may involve a fluorine group, an alcohol residue having 1 to 22 carbon atoms, a carboxylic acid residue having 1 to 22 carbon atoms, an aryl group having 6 to 24 carbon atoms, a polymer of alkylene oxide having 2 to 4 carbon atoms, or an alkylene oxide polymer residue in which a terminal of an alkylene oxide polymer having 2 to 4 carbon atoms is esterified with a fatty acid having 2 to 22 carbon atoms, or etherified with a higher alcohol having 2 to 22 carbon atoms or an aryl group having 6 to 24 carbon atoms. The letters m and n are integers of 2 or more.

Examples of the alkyl group having 1 to 22 carbon atoms that may contain a fluorine group include a methyl group, an ethyl group, a propyl group, an isopropyl group, an n-butyl group, an isobutyl group, a sec-butyl group, a tert-butyl group, a pentyl group, a hexyl group, an octyl group, a nonyl group, a decyl group, a dodecyl group, a tetradecyl group, a hexadecyl group, a stearyl group, an oleyl group and a behenyl group, etc., and at least one of them is used.

Examples of the alcohol residue having 1 to 22 carbon atoms include a methoxy group, an ethoxy group, a propoxy group, an isopropoxy group, a butoxy group, an isobutoxy group, a sec-butoxy group, a tert-butoxy group, a pentoxy group, a decanol residue, a dodecanol residue, a palm alcohol residue, a stearyl alcohol residue, an oleyl alcohol residue and a behenyl alcohol residue, etc., and at least one of them is used.

Examples of the carboxylic acid residue having 1 to 22 carbon atoms include an acetyl group, a propionic acid residue, a butanoic acid residue, a pentanoic acid residue, a hexanoic acid residue, an octanoic acid residue, a decanoic acid residue, dodecanoic acid residue, a stearic acid residue, an oleic acid residue and a behenic acid residue, etc., and at least one of them is used.

Examples of the aryl group having 6 to 24 carbon atoms include a phenyl group, a benzyl group, an ethylphenyl group, an octylphenyl group, a nonylphenyl group and a stearylphenyl group, etc., and at least one of them is used. Examples of the alkylene oxide polymer having 2 to 4 carbon atoms include homopolymer of ethylene oxide, propylene oxide and butylene oxide, or addition polymer of a mixture of at least two of them, and the degree of polymerization (n) thereof is 1 to 5,000.

Examples of the silicone oil include dimethyl polysiloxane, ethyl methyl polysiloxane, diethyl polysiloxane, methyl-hydrogen-polysiloxane, methyl phenyl polysiloxane, polyether modified organo polysiloxane such as dimethyl siloxane-methyl(polyoxyethylene) siloxane copolymer and dimethyl siloxane-methyl(polyoxyethylene-polyoxypropylene) siloxane copolymer, cyclic dimethyl polysiloxane such as dimethylsiloxane-alcoxy (having 4 to 12 carbon atoms)metylsiloxane copolymer, octamethyl cyclotetrasiloxane, decamethyl cyclopentasiloxane, dodecamethyl cyclohexasiloxane, fluorine modified organo polysiloxane such as fluoromethylsiloxane· dimethylsiloxane copolymer, fluoroalkyl· polyoxyalkylene modified organo polysiloxane such as fluoromethyl siloxane· polyoxyethylene methylsiloxane copolymer and fluoromethylsiloxane· polyoxyethylene polyoxypropylene methylsiloxane copolymer, terminal or side chain modified organo polysiloxane such as dimethyl polysiloxane modified substance in which a hydroxyl group is introduced in a terminal thereof, and hydroxymethylsiloxane· dimethylpolysiloxane copolymer in which a hydroxyl group is introduced in a side chain partially, and modified amino organo polysiloxane such as dimethyl amino butyl methylsiloxane· dimethylsiloxane polymer having a dialkyl aminoalkyl group in a side chain thereof, etc. Normally, the silicone oil having a viscosity of 100,000 (mPa· s: 25 °C) or less is selected as the component of the cosmetic composition. The content of such silicone oil ranges from 0.1 to 80 wt% (with respect to the total amount of the cosmetic composition), and the preferable content thereof ranges from 0.5 to 50 wt% (hereinafter, "wt%" will be referred to as --%--).

Examples of hydrocarbons include squalane, squalene, ceresin, paraffin, paraffin wax, liquid paraffin, pristane, polyisobutylene, micro crystallin wax, vaseline, etc. Examples of waxes include wax of bees wax, carnauba wax, candelilla wax, whale wax, etc., examples of animal oils include beef tallow, cattle leg grease, cattle bone fat, hardened beef tallow, hydrogenated oil, turtle oil, lard, horse grease, mink oil, liver oil, egg yolk oil, etc., examples of lanolin derivatives include lanolin, liquid lanolin, reduction lanolin, lanolin alcohol, hard lanolin, lanolin acetate, lanolin fatty acid isopropyl, POE lanolin alcohol ether, POE lanolin alcohol acetate, lanolin fatty acid polyethylene glycol, POE hydrogenation lanolin alcohol ether, etc., examples of fatty acids include lauric acid, myristic acid, palmitic acid, stearic acid, behenic acid, undecylenic acid, oleic acid, arachidonic acid, docosahexaenoic acid (DHA), isostearic acid, 12- hydroxy stearic acid, etc., examples of higher alcohol include lauryl alcohol, myristyl alcohol, palmityl alcohol, stearyl alcohol, behenyl alcohol, hexadecyl alcohol, oleyl alcohol, isostearyl alcohol, hexyl dodecanol, octyl dodecanol, cetostearyl alcohol, 2-decyl-tetra-decinol, cholesterol, phytosterol, sitosterol, lanosterol, POE cholesterol ether, mono stearyl glycerin ether (batyl alcohol), etc., examples of fatty acid esters include diisobutyl adipate, 2-hexyl decyl adipate, di-2- heptyl undecyl adipate, N-alkyl glycol monoisostearate, iso cetyl isostearate, trimethylolpropane triisostearate, ethylene glycol di-2-ethylhexanoate, cetyl 2-ethylhexanoate, trimethylolpropane tri-2-ethylhexanoate, pentaerythritol tetra -2-ethylhexanoate, cetyl ethylhexanoate, octyl dodecyl gum ester, oleyl oleate, octyl dodecyl oleate, decyl oleate, neopentylglycol dicaprate, triethyl citrate, ethylhexyl succinate, amyl acetate, ethyl acetate, butyl acetate, iso cetyl stearate, butyl stearate, diisopropyl sebacate, di-2-ethylhexyl sebacate, cetyl loctate, myristyl lactate, isopropyl palmitate, 2- ethylhexyl palmitate, 2- hexyl decyl palmitate, 2- heptyl undecyl palmititate, cholesteryl 12-hydroxystearate, di-pentaerythritol fatty acid ester, isopropyl myristate, octyl dodecyl myristate, 2- hexyl decyl myristate, myristyl myristate, hexyldecyl octanate, ethyl laurate, hexyl laurate, etc., examples of amino acid ester include N- lauroyl -L- glutamic acid -2- octyl dodecyl ester, etc., examples of organic acid ester include di-iso stearylmalate, etc., examples of glycerides include acetglyceride, glyceride tri-isooctanate, glyceride tri-isostearate, glyceride tri-iso palmitate, glyceride tri-2- ethylhexanoate, glyceride mono stearate, glyceride di-2- heptyl undecanoate, di-2- heptyl undecanoate, glyceride tri-myristate, etc., and examples of fluorinated hydrocarbon include polyperfluoroether, perfluorodecalin, perfluorooctane, etc.

Other components to be emulsified, which are to be used in the cosmetic composition in accordance with the present invention, include fatty acids such as lauric acid, myristic acid, palmitic acid, stearic acid, behenic acid, undecylenic acid, oleic acid, arachidonic acid, docosahexaenoic acid (DHA), isostearic acid, 12-hydroxystearic acid, etc., polyhydric alcohols including natural alcohols such as ethanol, isopropanol, rauryl alcohol, cetanol, stearyl alcohol, oleyl alcohol, lanoline alcohol, cholesterol, fitsterol,etc. and synthetic alcohols such as 2-hexyl decanol, isostearyl alcohole, 2-octyl dodecanol, etc., ethylene oxide, ethylene glycol, diethylene glycol, triethylene glycol, ethylene glycol monoethyl ether, ethylene glycol monobuthyl ether, diethylene glycol monomethyl ether, diethylene glycol monoethyl ether, polyethylene glycol, propylene oxide, propylene glycol, polypropylene glycol, 1,3-buthylene glycol, glycerin, pentaerythritol, sorbitol, mannitol, etc. These moisture retaining components are blended by properly selecting one or more kinds thereof, and the blending amount thereof depends on the kinds of the moisture retaining components so as not to be determined equally, but normally ranges from 0.5 to 20 %.

In the cosmetic composition of the present invention, the following nonionic surfactants, anionic surfactants, cationic surfactants and amphoteric surfactants can be blended as components thereof.

Examples of nonionic surfactants include polyoxyalkylene alkyl ether, polyoxyalkylene alkyl phenyl ether, polyoxyalkylene fatty acid ester, polyoxyalkylene sorbitan fatty acid ester, sorbitan fatty acid ester, polyoxyalkylene glycerin fatty acid ester, glycerin fatty acid ester, polyglycerin fatty acid ester, polyoxyalkylene hydrogenated castor oil, sucrose fatty acid ester, polyoxyalkylene alkyl amines, ethylene oxide· propylene oxide block copoymer, etc.

Polyoxyalkylene in the nonionic surfactants is composed of at least one of polyoxyethylene (hereinafter referred to as "POE"), polyoxypropylene (hereinafter referred to as "POP"), and polyoxybutylene (hereinafter referred to as "POB"). The polymerization mole number of each of POE, POP and POB is arbitrally determined based on the desired emulsion characteristics of the surfactant, and normally 3 to 200. In addition, the polymerization moler ratio of POE, POP and POB is arbitrally determined based on the desired emulsion characteristics of the surfactant. Preferably, polyoxyalkylene is composed of POE and POP, and a moler ratio of POE is 25 mole % or more.

Polyoxyalkylene alkylether having 2 to 4 carbon atoms is prepared by adding polyalkylene oxide to straight chain-type or branch type, saturated or unsaturated alcohol having 8 to 30 carbon atoms. More specifically, examples of polyoxyalkylene alkylether having 2 to 4 carbon atoms include POE (3 mole)octyl ether, POE(5 mole)dodecyl ether, POE(10 mole)oleyl ether, POE(15 mole)stearyl ether, POE(20 mole)behenyl ether, POE(10 mole) POP(10 mole)decyl ether, POE(15 mole)POP(2 mole)isostearyl ether, POE(10 mole)cholestanol ether, POE(0 mole)POP(0 mole) hydrogenerated lanolines, etc.

Polyoxyalkylene alkylpyenylether is prepared by adding polyalkylene oxide to straight chain-type or branch type alkylphenol and alkenylphenol, each having 1 to 22 carbon atoms. More specifically, examples of polyoxyalkylene alkylpyenylether include polyoxyethylene(3 mole)methylphenylether, POE(5 mole)octylphenylether, POE(10 mole)nonylphenyl ether, POE(15 mole)dodecylphenylether, etc.

Polyoxyalkylene fatty acid esters are prepared by adding polyalkylene oxido to straight chain-type or branch type satrurated fatty acid or unsaturated fatty acid having 8 to 22 carbon atoms. More specifically, examples of polyoxyalkylene fatty acid esters include POE(3 mole)octanic acid ester, POE(5 mole)decanic acid ester, POE(10 mole)dodecanic acid ester, POE(15 mole)stearic acid ester, POE(20 mole)behenylic acid ester, POE(15 mole)isostearic acid ester, POE(15 mole) POP(5 mole) oleic acid ester, etc.

Polyoxyalkylene sorbitan fatty acid esters are prepared by adding polyalkylene oxide to sorbitol and straight chain-type or branch type satrurated fatty acid or unsaturated fatty acid having 8 to 22 carbon atoms. More specifically, examples of apolyoxyalkylene sorbitan fatty acid esters include POE(5 mole)sorbitan monolaurate, POE(20 mole)sorbitan trilaurate, POE(20 mole)sorbitan monostearate, POE(20 mole)sorbitan sesquistearate, POE(20 mole)sorbitan tristearate, POE(20 mole)sorbitan monooleate, etc.

Sorbitan fatty acid esters are esters composed of sorbitol and straight chain-type or branch type saturated fatty acid or unsaturated fatty acid having 8 to 22 carbon atoms. More specifically, examples of sorbitan fatty acid esters include sorbitan monolaurate, sorbitan monopalmitate, sorbitan monostearate, sorbitan monoisostearate, sorbitan monooleate, sorbitan sesquioleate, sorbitan trioleate, penta-2-ethyl hexylic acid diglycerol sorbitan, tetra-2-ethyl hexylic acid diglycerol sorbitan, etc.

Polyoxyalkylene glycerin fatty acid esters are addition esters composed of glycerin, straight chain-type or branch type satrurated fatty acid or unsaturated fatty acid having 8 to 22 carbon atoms, and polyalkylene oxide. More specifically, examples of polyoxyalkylene glycerin fatty acid esters include POE(5 mole)glycerolmonolaurate, POE(10 mole)glycerolmonostearate, POE(15 mole)glyceroldistearate, POE(20 mole)POP(5 mole)glyceroldioleate, etc.

Polyoxyalkylene alkyl amines are prepared by adding polyalkylene oxide to first or secondary amine having 3 to 22 carbon atoms, and more specifically, examples thereof include POE(5 mole)didodecyl amine, diPOE(10)POP(3)dodecyl amine, POE(10 mole)distearyl amine, diPOE(10 mole)stearyl amine, diPOE(15 mole)oleyl amine, diPOE(17 mole)behenyl amine, etc.

Etylene oxide· propylene oxide copolymers are copolymers obtained by polymerizing ethylene oxide and propylene oxide in a moler ratio of 1:9 to 9:1 so as to have a molecular weight of about 500 to 50,000.

In addition, examples of anionic surfactants include fatty acid salts, alkyl sulfate salts, alkenyl sulfate salts, alkyl phenyl sulfate salts and alkenyl phenyl sulfate salts, alkyl phenyl polyoxyalkylene ether sulfate salts and alkenyl phenyl polyoxyalkylene ether sulfate salts, (di)alkyl sulfosuccinate salts, N-acylamino acid salts (acyl-N-methyl taurine), alkylbenzenesulfonate salts, alkylnaphtalenesulfonate salts, formaldehyde polycondensation of naphthalenesulfonate salts, etc. Preferable examples of metallic salts include sodium salts, potassium salts and ammonium salts.

Fatty acid salts are metallic salts of straight chain-type or branch type saturated fatty acid or unsaturated fatty acid having 8 to 30 carbon atoms, and, more specifically, examples thereof include sodium octylate, sodium decanoate, sodium dodecanoate, sodium tetradecanate, sodium stearate, sodium isostearate, sodium oleate, sodium linolenate, sodium edetate, etc.

Alkyl sulfates and alkenyl sulfates are straight chain-type or branch type satrurated or unsaturated Alkyl sulfates and alkenyl sulfates having 8 to 30 carbon atoms, and, more specifically, examples thereof include sodium octylsulfate, sodium decyl sulfate, sodium dodecyl sulfate, palm sodium alkyl sulfate, sodium stearyl sulfate, potassium isostearyl sulfate, ammonium oleyl sulfate, ammonium behenyl sulfate, etc.

Alkyl phenyl polyoxyalkylene ether sulfates and alkenyl phenyl polyoxyalkylene ether sulfates are sulfuric ester salts of addition product of phenyl group having alkyl group or alkenyl group, which has 1-22 carbon atoms and straight chains or branch chains,and polyoxyalkylene glycol having 2 to 4 carbon atoms, and, more specifically, examples thereof include tosyl POE(3 mole)sodium sulfate, octyl phenyl POE(5 mole)sodium sulfate, nonyl phenyl POE(10 mole)potassium sulfate, decyl phenyl POE (10 mole) sodium sulfate, octadecenyl phenyl POE(15 mole) potassium sulfate, isooctadecyl phenyl POE(15 mole)POP(5 mole) potassium sulfate, etc.

Examples of (di)alkyl sulfosuccinates salts include sodium dioctyl sulfosuccinate, di-2-ethylhexyl sulfo sodium succinate, monolauroyl monoethanol amido polyoxyethylene sulfo sodium succinate, lauryl polypropylene glycol sulfo sodium succinate, etc.

N-acylamino acid salts are acyl-N-methyl taurines, and more specifically, examples thereof include higher fatty acid amido sulfonate salts such as lauroyl sarcosine sodium, N-myristoyl-N-metyl taurine sodium, coconut fatty acid methyl taurine sodium, lauryl methyl taurine sodium, etc, and N-acyl glutamate such as N-lauroyl monosodium glutamate, N-stearoyl disodium glutamate, N-myristyl-L-monosodium glutamate, etc. Examples of alkylbenzenesulfonate salts include sodium dodecylbenzenesulfonate, potassium dodecyl benzenesulfonate, ammonium dodecylbenzenesulfonate, etc. These substances can be used alone or in combination of two or more of them.

Examples of cationic surfactant include amino acids, alkyl amine salts, fourth ammonium salts, pyridium salts, etc.

Examples of amino acids include lecithin of egg yolk or soybean, or lecithin derivative such as hydrogenated lecithin, lecithin hydroxide, etc.

Alkyl amine salts are salts of first or second amine having 3 to 22 carbon atoms, and carboxylic acid having 1 to 22 carbon atoms or inorganic mineral acid. And examples thereof include dodecylamine acetate salt, dodecylamine hydrochloride salt, dodecylamine stearate salt, dimetylamine stearate salt, etc.

Fourth ammonium salts are salts of fourth amine having 3 to 22 carbon atoms, and carboxylic acid having 1 to 22 carbon atoms or inorganic mineral acid. Examples thereof include stearyl trimethylammonium chloride, lauryl trimethylammonium chloride, distearyl dimethylammonium chloride, benzalconium chloride, benzetonium chloride, bromide palm alkyl (10 to 14 carbon atoms) isoquinolinium salt, chloride dodecyl imidazoiium salt.

Examples of pyridinium salts include poly(N,N-dimetyl-3,5-methylene piperidinium)chloride, cetylpyridinium chloride, etc.

Other examples of cationic surfactant include amine oxides such as dodecyl dimethylamine oxide, cationic polymer such as acrylic acid β -N-N dimethyl-N-ethyl ammonioetylic acid vinylpyrrolidone copolymer, etc.

Examples of ampholytic surfactant include betaines, phosphobetaines, sulfobetaines, glycine betaines, imidazolium betaines, amine oxides. More specifically, examples of betaines include dodecyl dimethylamino acetate betaine, stearyl dimethyl acetate betaine, dodecanic acid amido propyl dimethylamino acetate betaine, etc. Examples of phosphobetaines include 2-(dimethyl dodecyl ammonio)propio phosphate, 2-(dimethyl dodecyl ammonio)-2-hydroxypropio phosphate, etc. Examples of sulfobetaines include dodecyl dimethylethyl ammonium ethosulfate, etc. Examples of glycine betaines include dodecyl di(aminoethyl)glycine, etc. Examples of imidazolium betaines include 2-undecyl-N-, N, N-(hydroxyethyl carboxymethyl)-2-imidazoline sodium, 2-cocoil-2-imidazolinium hydroxide-1-carboxyethyroxy2 sodium salt, 2-heptadecyl-N-carboxymethyl-N-hydroxyethyl imidazolinium betaine, etc.

The preferred examples of surfactant include sucrose fatty acid ester, glycerin fatty acid ester, polyglyceryl fatty acid ester, lecithin, and derivative thereof, and the more preferred examples include sucrose stearate, sucrose palmitate, sucrose myristate, sucrose laurate, sucrose eruciate, sucrose oleate, glyceryl monostearate, glyceryl oleate, condensed hydroxystearic acid polyglycerin ester, condenced ricinoleate polyglyceryl ester, lecithin, hydrogenated lecithin, and hydroxide lecithin. These substances can be used alone, or in combination of two or more of them.

It is more preferable that the above-described surfactant is sucrose fatty acid ester, glycerin fatty acid ester, polyglyceryl fatty acid ester, or lecithin derivative.

Sucrose fatty acid esters are esters of sucrose and straight chain-type or branch type saturated fatty acid or unsaturated fatty acid having 8 to 22 carbon atoms, and, more specifically, examples thereof include sucrose behenic acid ester, sucrose stearic acid ester, sucrose palmitic acid ester, sucrose myristic acid ester, sucrose lauric acid ester, sucrose erucic acid ester, sucrose oleic acid ester, etc.

Glycerin fatty acid esters are esters of glycerin and straight chain-type or branch type saturated fatty acid or unsaturated fatty acid having 8 to 22 carbon atoms, and, more specifically, examples thereof include glycerin monolauric acid, glycerin sesquilauric acid, glycerin trilauric acid, glycerin monostearic acid, glycerin sesquistearic acid, glycerin tristearic acid, glycerin monooleic acid, glycerin sesquioleic acid, glycerin trioleic acid, glycerin monocotton-seed oil fatty acid, glycerin erucic acid, α , α '- glycerin oleic acid pyroglutamic acid, ester of a mixture of saturated fatty acid having 8 to 12 carbon atoms and glycerin, ester of stearic acid, malic acid and glycerin, etc.

Further examples of the components to be dispersed include lake pigment, organic pigment, inorganic pigment such as colour pigment, white pigment, extender pigment, nacreous pigment, metallic luster pigment, glass flake pigment, metal coated inorganic pigment, resin pigment, high polymer powders, functional pigment, etc. One or more of these pigments is used.

There are two kinds of lake pigment. One is the pigment in which water-soluble dye is made water-insoluble as salts of calcium, etc. Examples thereof include red No.202, red No.204, red No.206, red No.207, red No.208, red No.220, etc. Another one is the pigment in which water-soluble dye is made water-insoluble with aluminum sulfate, zirconium sulfate, etc., and is adsorbed on alumina. Examples thereof include yellow No. 5, red No. 230, etc.

The organic pigment is composed of colored powder which does not have hydrophilic groups in its molecular structure, and does not dissolve in water, oil and solvents, and coloring powder and light resistance thereof is excellent. Examples thereof include red No.228 of azo pigment, red No.226 of indigo pigment, blue No.404 of phthalocyanine pigment, etc.

Examples of the inorganic pigment include iron oxides having different colors, such as red iron oxide, yellow iron oxide, black iron oxide, etc., ultramarine blue, milori blue, chromium oxide, chromium hydroxide, magnesium oxide, cobalt oxide, cobalt titanium oxide carbon black, manganese violet, cobalt violet, etc.

White pigment is used for coloring, covering or the like, and examples thereof include titanium dioxide and zinc oxide.

Titanium dioxide, sintered titanium dioxide, zinc oxide, sintered zinc oxide may be subjected to the surface treatment with normally well known methods such as the silica treatment, the treatment with silicon compounds such as dimethyl polysiloxane, methyl-dydrogen-polysiloxane, trimethyl siloxysilicate, etc., the treatment with furuolo compounds such as perfluoro polyether phosphate, perfluoro alkyl phosphate, fluorine modified silicone, etc., the treatment with metallic soap such as zinc laurate, etc., the treatment with amino acids such as N-long chain acyl amino acid, etc., the treatment with oils such as higher fatty acids, higher alcohols, esters, waxes, etc.

Extender pigment is used for maintaining configurations of products, controlling extensibility, adhesion and luster thereof, and adjusting color tone thereof, and examples thereof include mica-based pigment such as mica, muscovite, synthesis mica, phlogopite, red mica, biotite, lithia mica, etc., clay minerals such as sericite, talc, kaolin, montmorillonite, zeolite, etc., synthetic inorganic powders such as magnesium carbonate, calcium carbonate, silicic acid, anhydrous silicic acid, aluminum silicate, magnesium silicate, magnesium aluminum silicate, sulfur-containing aluminum silicate, calcium silicate, barium silicate, strontium silicate, aluminum oxide, barium sulfate, etc.

The nacreous pigment is the pigment to be used for giving pearl-like luster, rainbow colors, and metallic feeling to products, and examples thereof include titanium diosxide covering mica, fishes scale foil, bismuth oxychloride, etc. In addition, pigment covered with iron oxide instead of titania, pigment having a titanate covering layer covered with another pigment of a transparent different color, etc. are used.

Examples of metallic luster pigment include aluminum powder, brass powder, copper powder, tin powder, gold dust, silver powder, etc., and colored metallic powder pigment prepared by coloring these metallic powders.

Glass flake pigment is covered with flake-shaped glass.

Metal-coated inorganic pigment is an inorganic pigment which is covered with metal and/or metal oxide by metal evaporation, and examples thereof include iron oxide covering aluminum, iron oxide covering mica, aluminum- manganese covering micaceous iron oxide, etc.

Resin pigment is composed of resin flake prepared by coloring resin film and cutting the colored resin film into flakes, examples of resin pigment include polyester film powder, polyethylene terephthalate·aluminum·epoxy laminate film powder, polyethylene terephthalate-polyolefine lamination film powder, polymethyl methacrylate, polyethylene terephthalate·polymethyl methacrylate lamination powder, nylon powder, etc.

Examples of functional pigment include boron nitride, synthetic boron gold phlogopite, photochromic pigment, composite fine-grain powder, etc.

The shape of these brightening pigments in accordance with the present invention is not limited specifically, and may be arbitrarily selected from grain shape, sheet shape, rod shape, etc. in accordance with the object and kind of pigment to be used. The size of pigment is not limited specifically, and may be arbitrally selected in accordance with the object and kind of pigment to be used. Normally, in the case of grain shape, the pigment having a mean particle diameter ranging from 0.01 µm to 5000µ m is used, and in the case of flake shape or rod shape, the pigment having a longer particle diameter ranging from 0.1 µm to 5000µ m is used.

The cosmetic compositions in accordance with the present invention include many kinds of cosmetic compositions according to their uses. Accordingly, if necessary, components to be blended in drugs, quasi-drugs, cosmetics, etc., such as purified water, hot spring water, deep sea water, thickening agents, coloring agents, moisture retaining agents, astringents, whitening agents, UV preventive agents, anti-inflammatory agents, skin (cell) activating agents, antibacterial agents, transdermal absorption promoting agents, carbonated agents, antioxidants, antiseptic agents, chelating agents, fade preventive agents, buffering agents, etc. may be added arbitrarily. The present invention does not limit the blending of these various additives provided that the effects of the invention are not damaged.

Examples of the thickening agents include natural high polymers such as gum arabic, guar gum, karaya gum, carrageenan, pectin, fucoidan, tragant gum, locust bean gum, galactomannan, xanthan gum, curdlan, gellant gum, fuco gel, casein, gelatine, starch, collagen, etc., semi-synthetic high polymers such as methyl cellulose, ethyl cellulose, methyl hydroxypropylcellulose, carboxymethylcellulose, hydroxymethylcellulose, hydroxypropylcellulose, sodium carboxymethylcellulose, alginic acid propylene glycol ester, etc., and synthetic high polymers such as polyvinyl alcohol, polyvinylpyrrolidone, carboxyvinyl polymer, polyacrylic acid, sodium polyacrylate, potassium polyacrylate, polyethyleneoxide, ethyleneoxidepropylene oxide copolymer, etc. Inorganic minerals such as bentonite, laponite, hectorite, etc. may be used together.

Examples of moisture retaining agents (components) include alkali simple spring water, deep sea water, mucopolysaccharides such as hyaluronic acid, chondroitin sulphate, dermatan sulfate, heparan sulfate, heparin, keratan sulfate, etc. or salts thereof, proteins such as collagen, elastin, keratin, derivatives thereof, and salts thereof, phospholipids derived from soybean and egg, glycolipids, ceramide, mucin, honey, erythritol, maltose, maltitol, xylitol, xylose, pentaerythritol, fructose, dextrine and derivatives thereof, saccharides such as mannitol, sorbitol, inositol, trehalose, glucose, etc., urea, asparagine, aspartic acid, alanine, arginine, isoleucine, orthinine, glutamine, glycine, glutamic acid, derivatives thereorf, and salts thereof, cysteine, cystine, citrulline, threonine, cerine, tyrosine, tryptophan, theanine, valine, histidine, hydroxylysine, hydroxyproline, pyrrolidone carbonate, salts thereof, amino acids such as proline, phenylalanine, methionine, lysine, derivaties thereof and salts thereof, D-panthenol, plant extract liquids. Examples of plant extract liquids include avocado extract, almond oil, carob extract, rice plant extract, strawberry extract, anise extract, pale red hollyhock extract, coptis extract, olive oil, lamium album extract, cacao butter, wild oat extract, ivy extract, sasa albo-marginata extract, gardenia extract, grapefruit extract, geranium thumbergii extract, great yellow gentian extract, burdock extract, tree peony vine extract, sesame extract, cactus extract, saponaria officinalis extract, ginger extract, rehmannia root extract, shear butter, spiraea extract, cnidium officinale extract, mallow extract, thymus vulgaris extract, camellia extract, corn extract, cordyceps sinensis extract, tormentila extract, houttuynia extract, ophiopogon tuber extract, lupinus perennis extract, hamamelis extract, mint extract, mentha viridis extract, peppermint extract, parsley extract, rose extract, sunflower extract, hinoki extract, looffah extract, prune extract, bucher's broom extract, borage oil, peony extract, jojoba oil, tilia miqueliana extracts, hop extract, pine extract, marronnier extract, macadamia nut oil, quince extracts, gromwell extracts, meadowfoam oil, melissa extracts, cornflower extracts, lily extracts, citron extracts, lime extracts, lavandula vera extract, gertianas scabra extract, sanguisorba extracts, apple extracts, etc. Examples of the moisturizing components further include yeast metabolite, yeast extraction extract, rice fermentation extract, fermented rice bran extract, euglena extract, lactic fermentation thing of raw milk and skim milk, trehalose derivatives thereof, alcohols and polyalcohols composed of natural alcohols such as ethanol, isopropanol, lauryl alcohol, cetanol, stearyl alcohol, oleyl alcohol, lanolin alcohol, cholesterol, phytosterol, etc., and synthetic alcohols such as 2-hexyl decanol, isostearyl alcohol, 2-octyl dodecanol, etc., ethylene oxide, ethylene glycol, diethylene glycol, triethylene glycol, ethylene glycol monoethyl ether, ethylene glycol monobutyl ether, diethylene glycol monomethyl ether, diethylene glycol monoethyl ether, polyethylene glycol, propylene oxide, propylene glycol, polypropylene glycol, 1,3-butylene glycol, glycerin, pentaerythritol, sorbitol, mannitol, etc. One or more of these moisture retaining agents can be selected properly and blended. The blending amount of the moisture retaining agents depends on the kind thereof so as not to be determined equally, but, normally, ranges from 0.5 to 20 %.

Examples of astringents (components) include zinc phenolsulfonate, sodium phenolsulfonate, and plant extracts. Examples of the plant extracts include arnica, hawthorn, cinchona, salvia, tilia miqueliana, ginseng, juniperus communis, rosemary, hypericum, ginkgo tree, melissa, ononis spinosa, marronnier, swertia japonica, garlic, chamomile, thyme, mint, nettle, red pepper, ginger, hop, western horse chestnut, Lavandula vera, carrot, leaf mustard, cinnamomum cassia, pine, cnidium officinale, elder, ostericum sieboldii, scopolia, peony, myrica, houttuynia, oryza sativa bone, bitter persimmon, calendula officinalis, corn poppy, gertiana scabra, grape, glehnia, orange, citron, iris, japanese summer orange, hamamelis, sweet clover, anise, japanese pepper, peony, eucalyptus, artemisia vulgaris indica, isodon japonicus, rice, sophora angustifolia, bread, clove, leaf of the walnut, scutellaria root, sage, tuberous roots of polygonum multiflorum, coptidis rhizoma, phellodendri cortex, scuttellaria baicalensis GEORGI. Houttuyniae herba, aurantii nobilis pericarpium, carrot, peony, codonopsitis radix, propolis, alisma rhizome, tannin, birch wood tar, royal jelly, yeast extract, etc.

One or more of these materials can be used in combination. Normally, the amount of the astringents ranges from 0.001 to 5 wt%, preferably, from 0.01 to 3 wt%, of the total amount of the cosmetic composition.

Examples of whitening agents (components) include tyrosinase inhibitor, endothelin antagonist, α -MSH inhibitor, glabridin, glablene, liquilitin, isoliquilitin, ellagic acid, ellagic acid salts, ellagic acid derivatives, kojic acid, kojic acid salts, kojic acid derivatives, arbutin, arubutin salts, arubutin derivatives, cysteine, cysteine salts, cysteine derivatives, vitamin C such as ascorbic acid, sodium ascorbate, ascorbyl stearate, ascorbyl palmitate, ascorbyl di-palmitate, magnesium ascorbyl-2-phosphate, vitamin C salts, vitamin C derivatives, glutathione, glutathione salts, glutathione derivatives, resorcin, resorcin salts, resorcin derivatives, rucinol, neo agarobiose, agarose oligosaccharide, and plant extracts. Examples of the plant extracts include asparagus extract, althaea officinalis extract, bistorta major extract, artemisiae capillaris flos extract, pea extract, dog rose fruit extract, scutellaria root extract, ononis spinosa extract, seaweed extract, firethorm extract, licorice extract, raspberry extract, sophora root extract, brown sugar extract, millettja reticulate extract, acanthopanacis cortex extract, wheat germ extract, asiasari radix extract, hawthorn extract, nomame herba extract, peony extract, lily extract, inula flower extract, mulberry bark extract, soybean extract, placenta extract, aralia elata extract, tea extract, angelica acutiloba extract moleasses extract, rosa polyantha extract, ampelopsis japonica extract, grape seed extract, beech extract, flor de manita extract, hop extract, rosa rugosae flos extract, chaenomelis fructus extract, saxifraga extract, coicis semen extract, rakanka extract, etc. One or more of these materials can be used properly. Normally, the blending amount of the whitening agents ranges from 0.01 to 10 %. Where the plant extracts, etc. are used as extracted liquids, the above blending amount is the amount converted into a dried solid extract.

Ultraviolet protective agents (components) include organic compounds-based ultraviolet absorbents and inorganic compounds-based ultraviolet ray scattering agents. Examples of ultraviolet absorbents include para-aminobenzoic acid -based ultraviolet absorbents, cinnamic acid-based ultraviolet absorbents, salicylic acid-based ultraviolet absorbents, benzophenone-based ultraviolet absorbents, etc. One or more of these absorbents is blended. Examples of para-aminobenzoic acid-based ultraviolet absorbents include para-aminobenzoic acid, glyceryl para-aminobenzoate, ethyl dihydro propyl para-aminobenzoate, amyl para dimethyl para-aminobenzoate, Octyl para methyl para-aminobenzoate, ethyl para-aminobenzoate, isobutyl para-aminobenzoate, etc., examples of cinnamic acid -based ultraviolet absorbents include isopropyl para methoxy cinnamate, diisopropyl cinnamic acid ester, octyl methoxy cinnamate, di-para methoxy cinnamate mono 2-ethylhexanoic acid glyceryl, etc., examples of salicylic acid-based ultraviolet absorbents include homo menthyl salicylate, octyl salicylate, phenyl salicylate, salicylic acid triethanolamine, amyl salicylate, benzil salicylate, p-tert butylphenyl salicylate, ethylene glycol salicylate, salicylic acid, etc., examples of benzophenone-based ultraviolet absorbents include dihydroxybenzophenone, tetrahydroxy benzophenone, oxybenzone, oxybenzone sulfonic acid, sodium hydroxy methoxybenzo phenon sulphonate, dihydroxy dimethoxybenzophenone, 2-hydroxy chlorobenzophenone, dioxybenzoone, sodium dihydroxy dimethoxybenzophenone disulphonate, 2-hydroxy-4-methoxy-4' methylbenzophenone, octabenzone, urocanic acid, ethyl urocanate, 4-tert-4'-methoxy dibenzoyl-methane, 2-(2'-hydroxy-5'-methylphenyl)benzotriazole, anthranilic acid, etc. Examples of the inorganic compounds to be used as the ultraviolet ray scattering agents include titanium oxide, zinc oxide, cerium oxide, zirconium oxide, iron oxide, etc.

Examples of antiinflammatory agents (components) include zinc oxide, sulfur, derivatives of sulfur, glycyrrhizin acid, derivatives and salts of glycyrrhizin acid, such as di-potassium glycyrrhigate and mono-ammonium glycyrrhigate, glycyrrhetic acid, derivatives and salts of glycyrrhetic acid such as β -glycyrrhetic acid, stearyl glycyrrhetinate, disodium 3-succinyloxyglycyrrhetinate, etc, tranexamic acid, chondroitin sulfate, mefenamic acid, phenylbutazone, indometacin, ibuprofen, ketoprofen, allantoin, guaiazulene, and derivatives and salts thereof, various kinds of microbes, animal extracts, plant extracts, etc.

Examples of skin (cell) activating agents (components) include deoxyribonucleic acid, salts of deoxyribonucleic acid, adenylic acid derivatives such as adenosine triphosphate, adenosine phosphate, salts thereof, ribonucleic acid, salts thereof, cyclic AMP, cyclic GMP, flavin adenine dinucleotide, guanine, adenine, cytosine, thymine, xanthine and derivatives threof, caffeine, theophylline and salts thereof, retinol, retinol derivatives such as retinol palmitate, retinol acetate, etc., retinal, retinal derivatives such as dehydroretinal, etc., carotene, vitamin A such as carotenoid, etc., thiamine, thiamine salts such as thiamine hydrochloride, thiamine sulfate, etc., riboflavin, riboflavin salts such as riboflavin acetate, etc., pyridoxine, pyridoxine salts such as pyridoxine hydrochloride, pyridoxine di-octanoate, etc, flavin adenine dinucleotide, cyanocobalamin, folic acids, nicotinic acid, nicotinic acid derivatives such as nicotinamide, nicotinic acid benzil, etc., vitamin B such as choline, etc., y - linolenic acid and derivatives thereof, eicosapentaenoic acid and derivatives thereof, estradiol and derivatives and salts thereof, organic acids such as glycolic acid, succinic acid, lactic acid, salicylic acid, etc. and derivatives and salts thereof.

Examples of antibacterial agents (components) include benzoic acid, sodium benzoate, carbolic acid, sorbic acid, potassium sorbate, para oxybenzoic acid ester, para chloro metacresol, hexachlorophene, benzalkonium chloride, chlorhexidine chloride, trichlorocarbanilide, quaternum, bis (2- pyridylthio-1-oxide)zinc, phenoxyethanol, thianthol, isopropyl methylphenol, etc.

Examples of antioxidants (components) include vitamin A such as retinol, dehydroretinol, retinyl acetate, retinyl palmitate, retinal, retinoic acid, vitamin A oil, derivatives and salts thereof, carotenoids such as α - carotene, β - carotene, Y - carotene, cryptoxanthin, astaxanthin, fucoxanthin, etc. and derivatives and salts thereof, vitamin B such as pyridoxine, pyridoxal, pyridoxal - 5 - phosphate ester, pyridoxamine, etc., and derivatives and salts thereof, vitamin C such as ascorbic acid, sodium ascorbate, ascorbyl stearate, ascorbyl palmitate, ascorbyl dipalmitate, ascorbic acid magnesium phosphate, etc. and derivatives and salts thereof, vitamin D such as ergocalciferol, cholecalciferol, 1,2,5 - dihydroxy - cholecalciferol, etc., derivatives and salts thereof, vitamin E such as α - tocopherol, β - tocopherol, γ - tocopherol, δ -tocopherol, α -tocotrienol, β -tocotrienol, γ - tocotrienol, δ - tocotrienol, tocopherol acetate, tocopherol nicotinate, etc., derivatives and salts thereof, tororocks(water-soluble derivatives of vitamine E), derivatives and salts thereof, dihydroxytoluene, butylhydroxytoluene, butylhydroxyanisol, dibutylhydroxytoluene, α -lipoic acid, dehydrolipoic acid, glutathione, derivatives and salts thereof, uric acid, erythorbic acid, derivatives and salts thereof such as sodium erythorbate,etc., gallic acid, derivatives and salts thereof such as propyl gallate,etc., rutin, derivatives and salts thereof such as α -glycosyl-rutin, etc., tryptophan, derivatives and salts thereof, histidine, derivatives and salts thereof, derivatives and salts of cysteine such as N -acetylcysteine, **N** - acetylhomocysteine, **N** - octanoilcysteine, **N -** acetylcysteinemethylester, etc. derivatives and salts of cystine such as , **N , N'** - diacetylcystinedimethylester, **N** , **N** ' - dioctanoilcystinedimethylester, **N** , **N** ' - dioctanoilhomocystinedimethylester, etc., carnosine, derivatives and salts thereof, homocarnosine, derivatives and salts thereof, anserine, derivatives and salts thereof, calcinine, derivatives and salts thereof, derivatives and salts of dipeptide or tripeptide containing histidine and/or triptophan and/or histamine, flavonoids such as flavanone, flavone, anthocyanin, anthocyanidin, flavonol, quelcitron, myricetin, phycetin, hamamelis tannin, catechin, epicatechin, gallocatechin, epigalocatechin, epicatechingallate, epigallocatechingallate, etc., tannic acid, caffeic acid, ferulic acid, protocatechuic acid, chalcone, oryzanol, carnot sole, sesamole, sesamin, sesamolein, zingerone, curcumin, tetrahydro curcumin, chlobamide, deoxychlobamide, shoga-ol, capsaicin, vanillylamide, ellagic acid, bromphenol, flavoglassine, melanoidin, riboflavin, riboflavin butylester, flavinmononucleotide, flavin adenine nucleotide, ubiquinone, ubiquinol, mannitol, bilirubin, cholesterol, ebselen, selenomethionine, ceruloplasmin, transferrin, lactoferrin, albumin, bilirubin superoxide dismutase, catalase, glutathione peroxidase, metallothionein, O-phosphono-pyrid oxylidene rhodamine, N - (2 - hydroxybenzyl) amino acid, derivatives and salts thereof, N - (4 - pyridoxyl methylene)amino acid, derivatives and salts thereof, which are disclosed in U.S.P. No.5,594,012, etc. The content of the antioxidants depends on the kind thereof so as not to be determined equally. But, normally, the content thereof ranges from 0.01 to 10 %. Where the plant extract, etc. are used as extracted liquids, the above-described content is the amount converted into a dried solid extract.

Examples of perfumes (components) include natural perfumes and synthetic perfumes. Examples of natural perfumes include natural plant perfumes such as rose oil, jasmine oil, neroli oil, lavender oil, tuberose oil, ylang ylang oil, clary sage oil, clove oil, peppermint oil, geranium oil, pachouli oil, sandlwood oil, cinnamon oil, coriander oil, nutmeg oil, pine oil, vanilla oil, balsam Peru oil, banana oil, apple oil, fennel oil, tonca beans oil , pepper oil, lemon oil, orange oil, bergamot oil, opopanax oil, vetiver oil, iris oil, oakmoss oil, anise oil, bois de rose oil, etc., and natural animal perfumes such as musk oil, civet oil, castoreum oil, ambergris oil, etc.

Examples of the synthetic perfume include limonene, β - caryophylene, cis -3- hexenol, linalool, farnesol, β - phenylethyl alcohol, geraniol, citronellol, terpineol, menthol, santalol, bacdanol, puramanol, lyranol, lilial, 2, 6-nonadienal, citral, α - hexyl cinnamic aldehyde, β - ionone, *1* - carvone, cyclopenta decanone, damascone, methyl ionone, irone, ISO E-super, acetyl cedrene, muscone, benzyl acetate, methyl dihydrojasmonate, methyl jasmonate, linalyl acetate, benzyl benzoate, γ - undecalactone, jasmine lactone, cyclopentadecanolide, ethylene brassylate, galactsolid, ambroxane, rose oxide, eugenol, indole, phenylacetaldehydedimethylacetal, aurnriol (Schiff base), schiff base, etc. Normally, a plurality of these perfumes are used in combination as a mixture perfume, as required.

Examples of the organic solvents (components) include ethanol, acetone, ethyl acetate, butyl acetate, 1,3-butylene glycol, ethylene glycol, diethylene glycol, triethylene glycol, polyethylene glycol, propylene glycol, dipropylene glycol, glycerin, butanol, propanol, etc.

Examples of sequestering agents and antiseptic agents include hidroxyethane diphosphonic acid salts, phenacetine, EDTA & salts thereof, parapenes, stannates, etc., and examples of high molecular compounds include poly(dimethyl allyl ammonium halide)type cationic polymer, tallowyl amine condensation product type cationic polymer obtained from polyethylene glycol, epichlorohydrin, propylene amine and tallow fatty acid, cocoyl amine condensation product type cationic polymer obtained from polyethylene glycol, epichlorohydrin, propylene amine and coconut oil fatty acid, copoymer type cationic polymer of vinyl pyrrolidone and dimethyl aminomethaacrylate, guaternary nitrogen-containing cellulose ether type cationic polymer, etc.

Examples of the pH adjusting agents include organic acids such as citric acid, malic acid, acetic acid, lactic acid, oxalic acid, tartaric acid, formic acid, levulinic acid, etc. and inorganic acids such as phosphoric acid, hydrochloric acid, etc.

Hereinafter, the producing method of five kinds of polysaccharides (A-1 to A-3) to be used in embodiments will be explained.

### 1 A-1: Polysaccharides of a product of a microorganism of Alkaligenes latus strain B-16 (rough product)

40.0 g of glucose [Wako Pure chemical Industries, Ltd., regent], 4.0 g of dipotassium hydrogen phosphate [Wako Pure chemical Industries, Ltd., regent], 2.0 g of potassium dihydrogen phosphate [Wako Pure chemical Industries, Ltd., regent], 0.1 g of sodium chloride [Wako Pure chemical Industries, Ltd., regent], 0.2 g of magnesium sulfate [Wako Pure chemical Industries, Ltd., regent], 1.0 g of potassium nitrate [Wako Pure chemical Industries, Ltd., regent], and 1.5 g of yeast extrat [OXOID CO. Ltd.] were dissolved in an ion exchange water, and an obtained aqueous solution was adjusted to a pH of 6.5 using sodium hydroxide or sulphuric acid so that the total volume is 1 liter. 150 mL of the obtained aqueous solution was transferred into a 500 mL conical flask and sterilized by autoclaving at 121 °C for 15 minutes. Then, the temperature of the solution was lowered to room temperature, and Alkaligenes latus strain B-16 (FERM BP-2015) was inoculated in the solution in the flask. And the solution was subjected to shaking culture at 30 °C for 6 days(180 rpm). After cultivation, about three volumes of isopropyl alcohol was added thereto and stirred for mixing them. A resultant precipitated agglomeration was filtered, recovered and dried under a reduced pressure to obtain polysaccharides of a product of a microorganism of Alkaligenes latus strain B-16 (A-1). The obtained polysaccharides include polysaccharide composed of fucose, glucose, glucuronic acid and rhamnose in a moler ratio of 1:2:1:1, as a main component, and another polysaccharide composed of fucose and mannose in a moler ratio of 1:1. The ratio of the former polysaccharide and the latter polysaccharides is 7:1 (weight ratio). The polysaccharides were hydrolyzed with sulfuric acid, and resultant constituting monosaccharides were analyzed with a high speed liquid chromatography (HPLC).

### 2 A-2: purifed product of above A-1

An aqueous solution of 0.5 wt% of polyssacharides: A-1 was prepared, and an aqueous solution of sodium hydroxide was added thereto to a pH of 12. The obtained aqueous solution was processed using columns of ion exchange resin "DIAON HPA-75(OH-)(brand name)"(manufactured by Nippon Rensui, CO.) at 8Ru or less, and filtered with a filtration auxiliary "Radiolight RL700" and a membrane filter of 5 µ m to remove proteins, nucleic acids, and microbes. After the filtered liquid was adjusted to a pH of 7 with dilute hydrochloric acid, the pressure of the liquid was reduced, and the liquid was concentrated. Then the polysaccharides were precipitated by using acetone, and washed with ten volumes of acetone to obtain polysaccharides (A-2) composed of fucose, glucose, glucuronic acid and rhamnose in the ratio of 1:2:1:1 and having a high molecular weight of fifty million.

### 3. A-3: Alcasealan (manufactured by Hakuto CO., LTD.)

Silicone oil used in the embodiments are listed, as follows.
dimethyl polysiloxane (viscosity 50 mPa·s, 25°C, made by Shin-Etsu Chemical Co., Ltd.)
dimethyl polysiloxane (viscosity 100 mPa·s, 25°C, made by Shin-Etsu Chemical Co., Ltd.)
dimethyl polysiloxane (viscosity 50,000 mPa·s, 25°C, made by Shin-Etsu Chemical Co., Ltd.)
methylphenyl polysiloxane (viscosity 500 mPa·s, 25°C, made by Nihonunica Corporation)
dimethyl polysiloxane· methyl(polyoxyethylene)siloxane copolymer (viscosity 1,600 cSt, " SH 3775C (trade name)" made by Toray Dow Corning Silicone Co. Ltd.

Next, emulsifying dispersants containing compact globules of polysaccharides of A-1 to A-3 as a main component were subjected to the emulsifying and dispersing performance test, and test data will be shown.

The above-described Alcasealan was used as the polysaccharide. When this Alcasealan is dispersed in water, it forms a network structure and becomes a sticky liquid so that the network structure must be formed into compact globules. To this effect, an aqueous solution of Alcasealan was prepared by dispersing Alcasealan powder in a predetermined amount of water, leaving it in this state for one day for swelling, and heating at 80 °C for 30 minutes. And urea was added the aqueous solution of Alcasealan to break hydrogen bonds of Alcasealan to form compact globules. In the case of Alcasealan being less than 0.1 wt%, compact globules were formed with an aqueous solution of urea with 4 mol/dm³ .

In order to examine whether aqueous dispersion liquids of Alcasealan, which are formed into compact globules, have an emulsifying performance against oils, similarly to that of normally available surfactants, the emulsifying performance was examined based on the dispersion concentration of Alcasealan using liquid paraffin as one of hydrocarbon oils. The examination results are shown in TABLE 1. As shown, liquid paraffin was emulsified to 70 wt% (water 30 wt%) with the aqueous dispersing liquid containing 0.05 wt% of Alcasealan. And the state of the solution has not changed after several days, and was very stable. Upon examining the emulsion states at various emulsifying temperatures ranging from 25 to 75 °C under the condition of 0.05 wt% of Alcasealan and 30 wt% of liquid paraffin being contained, the emulsion states were stable at every temperature.

**TABLE 1**

| | | Amount of liquid paraffin (wt%) | | | | |
|---|---|---|---|---|---|---|
| | | 10 | 30 | 50 | 60 | 70 |
| Amount of Alcasealan (wt%) | 0.03 | o | o | o | o | Δ |
| | 0.05 | o | o | o | o | o |
| | 0.09 | o | o | o | o | o |

Next, upon examining the emulsifying performance of Alcasealan at various concentrations of Alcasealsn under the condition of the concentation of liquid paraffin as oils being constantly 30 %, it has been clarified that Alcasealan can be emulsified when the concentration thereof is greater than 0.04 wt%.

Next, the emulsion states were examined using various kinds of oils under the condition of 0.04 wt% of Alcasealan and 30 wt% of oils being constantly contained. The examination results are shown in TABLE 2. In the examination, the oils are reagents manufactured by Kanto Chemical Co., Inc. and are hexadecane, silicone(S-H200;50 mPa· s manufactured by Dowchemical), isopropyl myristate, squalane, olive oil, jojoba oil, cetstearyl alcohol and oleyl alcohol. In a comparative example, oleic acid was used, and xantan gum (trade name "KELTROL" manufactured by CP Kelco) was used in place of Alcasealan.

**TABLE 2**

| | Oil | Emulsion Stability (after 1 month, room temperature) | Emulsion state |
|---|---|---|---|
| Embodiments | hexadecane | o | O/W type |
| | silicone | o | O/W type |
| | Isopropyl myristate | o | O/W type |
| | squalane | o | O/W type |
| | olive oil | o | O/W type |
| | jojoba oil | o | O/W type |
| | | o | O/W type |
| | Oleyl alcohol | o | O/W type |
| Comparative Example | oleic acid | x | O/W type |

These results show that Alcasealan has an excellent emulsifying performance, and resultant milky lotions are stable even with the concentration as low as 0.04 wt%. It has been considered that these results are caused by compact globules of Alcasealan adhering to a periphery of an oil drop to form an emulsifying dispersant phase and consequently, a three phase composed of water phase~ emulsifying dispersant phase~oil phase being formed on surfaces of emulsions.

Upon comparing the emulsifying method (three-phase emulsifying method) using the emulsifying dispersant which contains compact globules of Alcasealan and multi-branched polysaccharides as main components with the conventional emulsifying method using surfactants, the following features have been recognized.

First, in the conventional emulsifying method, the surfactants are adsorbed on interfaces between oil and water to decrease the surface energy therein, thereby performing emulsification, but in the three-phase emulsifying method, nanoparticles adhere to interfaces between oil and water with van der Waals' force to form an emulsifying dispersant phase so that the emulsification is possible without varying the surface energy, irrespective of required HLB values of oily bases to be emulsified.

As a result, in the conventional emulsifying method with the surfactants, incorporation of oil drops may occur due to the thermal collisions thereof, but in the three-phase emulsifying method, nanoparticles adhere to surfaces of oil drops as a emulsifier phase so that incorporation of oil drops is difficult to occur upon collisions thereof, whereby resultant emulsions can be stabilized against heat and for a long period of time.

In addition, in the conventional emulsifying method with the surfactants, suitable surfactants must be selected according to the properties of the oil drops, but in the three-phase emulsifying method, after selecting the nanoparticles, the same emulsifyer can be used irrespective of the kinds of the oil drops so that emulsions composed of different kinds of oils can coexist with each other and can be mixed together.

Furthermore, in the conventional emulsifying method, a large amount of surfactant has been required to form a microemulsion from oils, but in the three-phase emulsifying method, emulsification is possible using an emulsifying dispersant with a very small concentration.

In addition, with the above-described three-phase emulsion, 1)oil drops with a size as great as salmon roes can be stably formed, 2) a creaming caused by the difference in specific gravity has not changed the emulsion state thereof upon removing a continuous outer phase, and 3)a three-phase emulsification type emulsion can be formed even when additives are added to a water phase or an oil phase.

Next, embodiments 1 through 12 of the present invention, in which the above-described polysaccharides with particulate structures are used, will be explained.

Alcasealan of A-1 through A-3 must be formed into compact globules, and after a prescribed amount of Alcasealan was dispersed in water, a mechanically strong shearing force (8000 rpm for 20 minutes) was applied with a homogenizer (manufactured by IKA) to form compact globues.

### Embodiment 1

In the embodiment 1, lotion 1 was prepared in the following composition ratio.

| (No) | (Blending Components) | (wt%) |
|---|---|---|
| 1. | ethanol | 5.00 |
| 2. | glycerin | 3.00 |
| 3. | 1,3-butylene glycol | 3.00 |
| 4. | dipotassium glycyrrhizin | 0.20 |
| 5. | sucrose lauric acid ester (Cosmelike L-160, made by Dai-ichi Kogyo Seiyaku Co. Ltd.) | 0.50 |
| 6. | polysaccharide (A-1) | 0.02 |
| 7. | methylphenyl polysiloxane (B-4) | 2.00 |
| 8. | methyl paraminobenzoate | 0.10 |
| 9. | pure water | balance |

A mixture 1 was prepared by mixing the blending components Nos. 1 to 5. No.7 and No. 8. And by adding No. 6 to No. 9, and stirring at 8,000 rpm with a homomixer (manufactured by IKA) for 20 mitutes, compact globules were formed. Next, while stirring at 5,000rpm, No. 7 was added for emulsification. And the mixture 1 was added to prepare an emulsion as the lotion 1.

### Embodiment 2

In the embodiment 2, lotion 2 was prepared in the following composition ratio.

| (No.) | (Blending Components) | (wt%) |
|---|---|---|
| 1. | ethanol | 14.00 |
| 2. | glycerin | 4.00 |
| 3. | 1,3-butylene glycol | 2.00 |
| 4. | titanium oxide ("MF-100SAK" manufactured by TAYCA CORPORATION) | 0.05 |
| 5. | zinc oxide ("FUJI ZnO-SMS" manufactured by Fuji Pigment Co., Ltd.) | 0.50 |
| 6. | kaolin | 2.00 |
| 7. | methylphenyl polysiloxane (B-4) | 1.00 |
| 8. | polysaccharide (A-3) | 0.05 |
| 9. | ascorbic acid magnesium phosphate | 3.00 |
| 10. | citric acid | 1.00 |
| 11. | sodium hydroxide | proper quantity |
| 12. | methyl paraminobenzoate | 0.10 |
| 13. | deep seawater | balance |

A mixture 2 was prepared by mixing the blending components Nos. 1 to 3 with No. 12. After dissolving No. 10 in 1/4 part of No. 13, No. 9 was added while stirring. And pH was adjusted with No. 11 to pH6.5 to obtain a mixture 3.

And No. 8 was added to 4/5 parts of No. 13, and by stirring at 8,000rpm with the homomixer ((manufactured by IKA) for 20 minutes, compact globules were formed. And while stirring at 5,000 rpm, No. 7 was added for emulsification. And after adding the mixture 3, NO. 4, No. 5, and No. 6 were successively added, and the mixture 2 was added to prepare an emulsion as the lotion 2

### Embodiment 3

In the embodiment 3, milky lotion 1 was prepared in the following composition ratio.

| (No) | (Blending Components) | (wt%) |
|---|---|---|
| 1. | methylphenyl polysiloxane (B-4) | 10.00 |
| 2. | dimethyl polysiloxane (B-2) | 5.00 |
| 3. | isopropyle myristic acid | 5.00 |
| 4. | titanium dioxide | 5.00 |
| 5. | zinc oxide | 3.00 |
| 6. | sorbitan stearate | 0.50 |
| 7. | stearic acid | 0.50 |
| 8. | sucrose myristic acid ester | 1.00 |
| 9. | polysaccharide (A-2) | 0.02 |
| 10. | glycerin | 3.00 |
| 11. | 1,3-butylene glycol | 5.00 |
| 12. | methyl paraoxybenzoate | 0.10 |
| 13. | pure water | balance |

A mixture 4 was prepared by mixing the blending component No. 8 with the blending components Nos. 10, 11, 12 while stirring.

A mixture 5 was prepared by mixing the blending components Nos. 4 and 5 with the blending components Nos. 1 through 3.

A mixture 6 was prepared by adding the mixture 5 to the mixture 4 while stirring and mixing them.

And No. 9 was added to the blending component No. 13 and stirred at 8,000rpm with the homomixer (manufactured by IKA) for 20 minutes to form compact globules. And while stirring at 5,000 rpm, the mixture 6 was added for emulsification and dispersion. And after heating to 70 °C, NO. 6 and No. 7 which have been heated to 70 °C were successively added to prepare an emulsion, and the emulsion was mixed homogeneously and cooled to room temperature while stirring with a propeller stirrer, thereby obtaining a milky lotion 1.

### Embodiment 4

In the embodiment 4, milky lotion 2 was prepared in the following composition ratio.

| (No) | (Blending Components) | (wt%) |
|---|---|---|
| 1. | methylphenyl polysiloxane (B-4) | 8.00 |
| 2. | squalane | 2.00 |
| 3. | titanium dioxide | 5.00 |
| 4. | zinc oxide | 3.00 |
| 5. | iron oxide (iron red)(manufactured by TODA KOGYO CORP.) | 0.50 |
| 6. | iron oxide (yellow)(manufactured by TODA KOGYO CORP.) | 1.50 |
| 7. | iron oxide (black)(manufactured by TODA KOGYO CORP.) | 0.20 |
| 8. | sorbitan stearate | 0.50 |
| 9. | stearic acid | 0.50 |
| 10. | sucrose myristic acid ester | 1.00 |
| 11. | polysaccharide (A-2) | 0.10 |
| 12. | glycerin | 3.00 |
| 13. | 1,3-butylene glycol | 5.00 |
| 14. | methyl paraoxybenzoate | 0.10 |
| 15. | pure water | balance |

A mixture 7 was prepared by adding the blending component No. 10 to the blending components Nos. 12, 13 and 14 and mixing them while stirring. A mixture 8 was prepared by adding the blending components Nos. 3 and 4 to the blending components Nos. 1 and 2 and mixing them.

The mixture 8 was added to the mixture 7 while stirring and mixed together to prepare a mixture 9.

And No. 11 was added to the blending component No. 15 and stirred at 8,000rpm with the homomixer ((manufactured by IKA) for 20 minutes to form compact globules. And while stirring at 5,000 rpm, the mixture 9 was added for emulsification and dispersion. And NO. 5 through No. 7 were successively added and mixed together. After heated to 70°C, No. 8 and No. 9 which have been heated to 70°C, were successively added to prepare an emulsion, and the emulsion was mixed homogeneously and cooled to room temperature while stirring with a propeller stirrer, thereby obtaining a milky lotion 2.

### Embodiment 5

In the embodiment 5, milky lotion 3 was prepared in the following composition ratio.

| (No) | (Blending Components) | (wt%) |
|---|---|---|
| 1. | polyoxyethylene (10 mole addition) sorbitan monostearate | 1.00 |
| 2. | polyoxyethylene (60 mole addition) sorbitan trioleate | 0.50 |
| 3. | glyceryl monostearate | 1.00 |
| 4. | stearic acid | 0.50 |
| 5. | dimethyl polysiloxane (B-2) | 5.00 |
| 6. | squalane | 4.00 |
| 7. | isopropyl paramethoxycinnamate | 0.50 |
| 8. | polysaccharide (A-2) | 0.10 |
| 9. | ascorbic acid magnesium phosphate | 5.00 |
| 10. | citric acid | 1.50 |
| 11. | titanium dioxide | 5.00 |
| 12. | methyl paraoxybenzoate | 0.10 |
| 13. | carboxyvinyl polymer | 0.10 |
| 14. | sodium hydroxide | 0.05 |
| 15. | ethanol | 5.00 |
| 16. | pure water | balance |

A mixture 10 was prepared by mixing the blending components Nos. 1 to 3.

A mixture 11 was prepared by mixing the blending components Nos. 4, 7 and 12.

The mixture 11 was gradually added to the blending component 5 and mixed together, and No.6 was further added to obtain a mixture 12.

After dissolving No. 10 in 1/5 part of the blending component No. 16, No. 9 was added while stirring. And pH was adjusted with No. 14 to pH 6.5 to obtain a mixture 13.

And No. 8 was added to 4/5 parts of No. 16, and stirred at 8,000rpm with the homomixer (manufactured by IKA) for 20 minutes to form compact globules. And while stirring with a propeller stirrer, No. 13 was added and the temperature was raised to 70°C to dissolve it completely. Next, while stirring at 5,000 rpm, the mixture 13 was added, and the mixture 12 was speedily added for emulsification. Furthermore, the mixture 11 was added to prepare an emulsion, and after the emulsion was cooled naturally while stirring, No. 15 was added to obtain a milky lotion 3.

### Embodiment 6

In the embodiment 6, cream 1 was prepared in the following composition ratio.

| (No) | (Blending Components) | (wt%) |
|---|---|---|
| 1. | dimethyl polysiloxane (B-2) | 5.00 |
| 2. | glycerin monostearate | 2.00 |
| 3. | stearic acid | 2.00 |
| 4. | stearyl alcohol | 6.00 |
| 5. | hydrogenated lanolin | 4.00 |
| 6. | squalane | 9.00 |
| 7. | octyl dodecanol | 10.00 |
| 8. | dimethyl polysiloxane-methyl(polyoxyethylene) siloxane copolymer (B-6) | 5.00 |
| 9. | polysaccharide (A-3) | 0.05 |
| 10. | glycyrrhizinic acid | 0.20 |
| 11. | ascorbic acid magnesium phosphate | 6.00 |
| 12. | citric acid | 3.00 |
| 13. | sodium hydroxide | 0.20 |
| 14. | 1,3-butylene glycol | 4.00 |
| 15. | methyl paraoxybenzoate | 0.10 |
| 16. | pure water | balance |

A mixture 14 was prepared by mixing the blending components Nos. 2, 3, 10, 14 and 15.

The blending components Nos. 1, 4 through 8 were mixed, and heated to 70°C to dissolve them while stirring to obtain a mixture 15.

No. 12 was dissolved in 1/5 part of No. 16, and No. 11 was added while stirring. The pH was adjusted to pH6.5 with No. 13 to prepare a mixture 16.

And No. 9 was added to 4/5 parts of No. 16, and stirred at 8,000rpm with the homomixer (manufactured by IKA) for 20 minutes to form compact globules. And while stirring with a propeller stirrer, the mixture 16 was added and the temperature was raised to 70°C. Next, while stirring at 5,000 rpm, the mixture 15 which had bee heated to 70°C was added for emulsification. And the mixture 14 was added to prepare an emulsion, and the emulsion was cooled naturally while stirring to obtain a cream 1.

### Embodiment 7

In the embodiment 7, foundation cream 1 was prepared in the following composition ratio.

| (No) | (Blending Components) | (wt%) |
|---|---|---|
| 1. | lanolin | 7.00 |
| 2. | dimethyl polysiloxane (B-2) | 5.00 |
| 3. | isopropyle myristic acid | 2.00 |
| 4. | cetanol | 1.00 |
| 5. | 2-ethylhexyl paramethoxy cinnamate | 3.00 |
| 6. | 4-t-butyl-4'-methoxydibenzoyl methane | 1.00 |
| 7. | sorbitan fatty acid ester | 0.50 |
| 8. | glycerin | 5.00 |
| 9. | triethanol amine | 1.00 |
| 10. | carboxymethyl cellulose (trade name: CMC Daicel 1150, made by Daicel Chemical Industries, Ltd.) | 0.10 |
| 11. | mica (trade name: MICA Y-3000, made by Yamaguchi-Mica Co. Ltd.) | 15.00 |
| 12. | talc (trade name: Victory Light SK-A, made by Shokosan Laboratories CO.) | 6.00 |
| 13. | iron oxide | 6.50 |
| 14. | Nylon powder (trade name: Amilan, made by Toray Industries, Inc.) | 2.00 |
| 15. | silica (trade name Sun-sphere H-31, made by Asahi Glass Company) | 0.60 |
| 16. | polysaccharide (A-3) | 0.05 |
| 17. | Extract of seeds of coix lachryma jobi (made by Maruzen Pharmaceuticals Co., Ltd.) | 0.50 |
| 18. | phenoxy ethanol | 0.10 |
| 19. | pure water | balance |

A mixture 17 was prepared by stirring the blending components Nos. 1 to 4 to mix them with each other, and heating them to 70°C for dissolving.

A mixture 18 was prepared by dissolving the blending component No. 7 in the blending component No. 8 and stirring, mixing and dispersing the blending components Nos. 11 to 15.

A mixture 19 was prepared by stirring and mixing the blending components Nos. 5, 6, 9, 17 and 18.

And the No.16 was added to the blending component No. 19, and stirred at 8,000 rpm with a homomixer(manufactured by IKA) for 20 minutes to form compact globules. And while stirring with a propeller stirrer, the blending component No. 10 was added and the temperature was raised to 70°C. Next, while stirring at 5,000 rpm, the mixture 15 which had bee heated to 70°C was added for emulsification.

The mixture 18 and the mixture 19 were successively added and mixed together, and cooled to room temperature while stirring with a propeller stirrer to obtain a foundation cream 1.

### Embodiment 8

In the embodiment 8, hair rinse 1 was prepared in the following composition ratio.

| (No) | (Blending Components) | (wt%) |
|---|---|---|
| 1. | trimethyl ammonium chlorostearate | 1.0 |
| 2. | cetanol | 3.0 |
| 3. | dimethyl polysiloxane (B-2) | 8.0 |
| 4. | polyoxyethylene (12 mole addition ) stearyl ether | 1.0 |
| 5. | propylene glycol | 5.0 |
| 6. | polysaccharide (A-3) | 0.1 |
| 7. | methyl paraoxybenzoate | 0.1 |
| 8. | potassium chloride | 0.3 |
| 9. | citric acid | 0.2 |
| 10. | perfume | proper quantity |
| 11. | pure water | balance |

A mixture 20 was prepared by mixing the blending components Nos. 1, 4, 5, 7, 8, 9 and 10.

And No. 6 was added to the blending component No. 11, and stirred at 8,000 rpm with a homomixer(manufactured by IKA) for 20 minutes to form compact globules. And they were heated to 70°C while stirring at 5,000 rpm, and then, the blending components Nos. 2 and 3 are added thereto for emulsification. Next, the mixture 20 was added to prepare an emulsion, and cooled to room temperature while stirring to obtain a hair rinse 1.

### Embodiment 9

In the embodiment 9, face powder 1 was prepared in the following composition ratio.

| (No) | (Blending Components) | (wt%) |
|---|---|---|
| 1. | glycerin | 4.00 |
| 2. | 1,3-butylene glycol | 4.00 |
| 3. | methyl phenyl polysiloxane | 5.00 |
| 4. | perfume | 0.01 |
| 5. | polyoxyethylene (23 mole addition )lauryl ether | 0.20 |
| 6. | iron oxide (red) | 0.50 |
| 7. | iron oxide (yellow) | 1.40 |
| 8. | iron oxide (black) | 0.20 |
| 9. | kaolin | 5.00 |
| 10. | titanium dioxide | 5.00 |
| 11. | propyl paraoxybenzoate | 0.10 |
| 12. | polysaccharide (A-3) | 0.06 |
| 13. | pure water | balance |

A mixture 21 was prepared by stirring and mixing the blending components Nos. 2, 4, 5 and 11.

A mixture 22 was prepared by adding the blending components Nos. 6 to 10 to the blending component No 3 and mixing together to be dispersed.

And No. 12 was added to the blending component No. 13, and stirred at 8,000 rpm with a homomixer(manufactured by IKA) for 20 minutes to form compact globules. And the mixture 24 were added thereto while stirring at 5,000 rpm for emulsification. Next, the mixture 21 was added and stirred to obtain a face powder 1.

### Embodiment 10

In the embodiment 10, eyeliner 1 was prepared in the following composition ratio.

| (No) | (Blending Components) | (wt%) |
|---|---|---|
| 1. | iron oxide (black) | 4.00 |
| 2. | titanium dioxide | 5.00 |
| 3. | pearl pigment | 0.20 |
| 4. | ethanol | 4.00 |
| 5. | glycerin | 6.00 |
| 6. | polyoxyethylene (100 mole addition ) hydrogenated caster oil | 0.50 |
| 7. | jojoba oil | 10.10 |
| 8. | polysaccharide (A-3) | 0.08 |
| 9. | phenoxy ethanol | 0.15 |
| 10. | pure water | balance |

A mixture 25 was prepared by adding the blending components Nos. 1 to 3 to the blending component No. 7 and mixing together to be dispersed.

A mixture 26 was prepared by stirring and mixing the blending components No. 6 and 9 with the blending component 5.

And No. 8 was added to the blending component No. 10, and stirred at 8,000 rpm with a homomixer(manufactured by IKA) for 20 minutes to form compact globules. And the mixture 25 was added thereto while stirring at 5,000 rpm for emulsification. Next, the mixture 26 was added and the blending component No. 4 was added and stirred to obtain an eye liner 1.

| (No) | (Blending Components) | (wt%) |
|---|---|---|
| 1. | kaolin | 10.00 |
| 2. | titanium dioxide | 2.00 |
| 3. | iron oxide (red) | 0.20 |
| 4. | #202 red | 0.30 |
| 5. | ethanol | 4.00 |
| 6. | glycerin | 4.00 |
| 7. | polyglycerin fatty acid ester | 0.50 |
| 8. | squalane | 5.00 |
| 9. | perfume | 0.02 |
| 10. | polysaccharide (A-3) | 0.10 |
| 11. | phenoxy ethanol | 0.15 |
| 12. | pure water | balance |

A mixture 27 was prepared by homogeneously dispersing the blending components Nos. 1 to 4 in the blending components Nos. 5 to 9 and a portion of the blending component No. 12. A mixture 28 was prepared by mixing and dissolving the blending components Nos. 10 to 12. The mixture 27 was added to the mixture 28 while stirring the mixture 28 homogeneously to obtain a cheek rouge 1

### [Stability test of cosmetic composition]

After sampling 200 ml of the cosmetic compositions in the embodiments 1 through 10, which were just after being prepared, in sample bottles, and measuring the viscosity of the compositions, the bottles were sealed, and placed in a thermostatic chamber at 45°C. Then, the viscosity was measured again after 12 weeks. 100 ml of the cosmetic compositions were sampled in 100 ml measuring cylinders, and the cylinders were sealed and placed in a thermostatic chamber at 45°C. After 12 weeks, the volume of an oil layer floating on each cosmetic composition in the 100 ml measuring cylinder, and the volume of an aqueous phase separated under the cosmetic composition were measured. The results as measured by the following evaluation criteria are shown in Table 3.

### (Evaluation critera of stability)

o : no separation and precipitation are observed by eyes; and
x: separation and precipitation are observed by eyes

### (Evaluation of feeling of uses)

The cosmetic compositions (A) just after preparation, and the cosmetic compositions (B) after being placed in the thermostatic chambers at 45°C for continuous 12 weeks, which were listed on TABLE 3, were respectively subdivided in ten sets of vessels, each having an identical external appearance to each other, such that they cannot be distinguished from each other. Two panelers in every ages from teens to fifties were selected. And ten panelers in total carried out sensory tests. A proper quantity of each of the cosmetic composition (A) except for the hair rinses 1 and 2 was extended on the backs of both hands of each paneler, and "stickiness" and "smoothness" thereof were respectively evaluated. The cosmetic composition (B) was also evaluated in the same manner. The hair rinse was evaluated by the feeling when each paneler touched her hair after using the hair rinse. The sample showing precipitation and separation was evaluated after shaking. The evaluation criteria of "Stickiness" and "smoothness" were as follows. The results are shown in Table 4.

### (Evaluation criteria of "stickiness")

○ : evaluated by eight or more out of ten panelers to have fresh feeling with little sticky feeling
Δ : evaluated by five to seven out of ten panelers to have fresh feeling with little sticky feeling
x: evaluated by four or less out of ten panelers to have fresh feeling with no sticky feeling

### (Evaluation criteria of "smoothness")

o : evaluated by eight or more out of ten panelers to have smooth feeling
Δ: evaluated by five to seven out of ten panelers to have smooth feeling
x: evaluated by four or less out of ten panelers to have smooth feeling

**TABLE 3**

| Example | Cosmetic composition | viscosity (mP a · s ) | | Separation and precipitation |
|---|---|---|---|---|
| | | just after preparation | after 12 weeks | |
| Embodiments | lotion 1 | 45 | 40 | o |
| | lotion 2 | 105 | 105 | o |
| | milky lotion **1** | 150 | 145 | o |
| | milky lotion 2 | 265 | 270 | o |
| | milky lotion 3 | 315 | 310 | o |
| | Cream **1** | 750 | 750 | o |
| | foundation cream **1** | 3265 | 3260 | o |
| | hair rinse **1** | 465 | 460 | o |
| | face powder **1** | 370 | 370 | o |
| | eyeliner **1** | 430 | 435 | o |
| | cheek rouge 1 | 525 | 525 | o |

**TABLE 4**

| Example | Cosmetic composition blended oil with silicone oil | stickiness | | smoothness | |
|---|---|---|---|---|---|
| | | Just after preparation | after 12 weeks | Just after preparation | after 12 weeks |
| Embodiments | lotion **1** | o | o | o | o |
| | lotion 2 | o | o | o | o |
| | milky lotion **1** | o | o | o | o |
| | milky lotion 2 | o | o | o | o |
| | milky lotion 3 | o | o | o | o |
| | cream **1** | o | o | o | o |
| | foundation cream **1** | o | o | o | o |
| | hair rinse **1** | o | o | o | o |
| | face powder **1** | o | o | o | o |
| | eyeliner **1** | o | o | o | o |
| | cheek rouge **1** | o | o | o | o |

### BRIEF EXPLANATION OF DRAWINGS

FIG. 1 is a diagram explaining the emulsification mechanism, and FIG. 1(A) is a diagram explaining the adsorption mechanism of a monomolecules film of a conventional surfactant, and FIG. 1(B) is a diagram explaining the adhesion mechanism of nanoparticles;
FIG. 2(A) is a diagram explaining the phenomena caused by thermal collision in a conventional adsorption monomolecules-type emulsion, and FIG. 2(B) is a diagram explaining the phenomena caused by thermal collision in an emulsifier adhesion-type emulsion; and

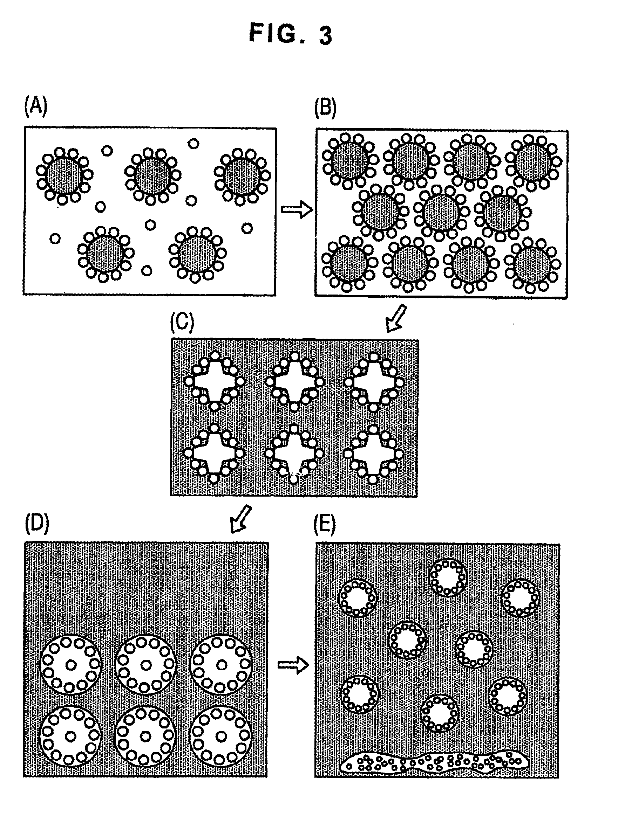

FIG. 3 is a model diagram showing the difference in emulsion state, resulted from the amount of an oil phase.

## Claims

1. A cosmetic composition **characterized in that** the cosmetic composition contains an emulsifying dispersant which includes polysaccharides with particulate structures as a main component, and also contains components to be emulsified.

2. A cosmetic composition as claimed in claim 1, which is **characterized in that** said emulsifying dispersant which includes said polysaccharides with particulate structures as a main component exists as a three-phase emulsifying dispersant which adheres to a periphery of said components to be emulsified like a layer to define an intermediate layer.

3. A cosmetic composition as claimed in one of claims 1 and 2, wherein said polysaccharides with particulate structures have an average particle diameter ranging from 8 nm to 500 nm.

4. A cosmetic composition as claimed in one of claims 1 through 3, wherein said polysaccharides with particulate structures are polysaccharides which are formed into compact globules.

5. A cosmetic composition as claimed in one of claims 1 through 4, wherein said polysaccharides with particulate structures contain at least one of fucose, glucose, glucuronic acid and rhamnose as a constituting monosaccharide, and contain fucose and/or rhamnose in side chains thereof.

6. A cosmetic composition as claimed in one of claims 1 through 5, wherein said polysaccharides with particulate structures contain at least the polysaccharide represented by the following formula (1).

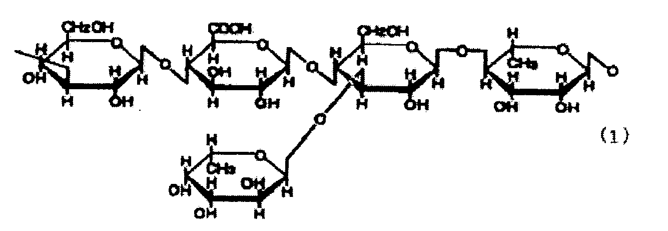

7. A cosmetic composition as claimed in one of claims 1 through 6, wherein said emulsifying dispersant which includes the polysaccharides with particulate structures as a main component exists in the weight ratio of from 1 : 50 to 1 : 1000 to said components to be emulsified.

8. A cosmetic composition as claimed in one of claims 1 through 7, wherein said emulsifying dispersant which includes the polysaccharides with particulate structures as a main component contains urea.

9. A cosmetic composition as claimed in one of claims 1 through 8, wherein said components to be emulsified are functional oily bases.

10. A cosmetic composition as claimed in one of claims 1 through 9, wherein one of said components to be emulsified is silicone oil.

11. A cosmetic composition as claimed in one of claims 1 through 8, wherein one of said components to be emulsified is titanium oxide particles and/or titanium oxide particles subjected to surface treatments.

12. A method for producing a cosmetic composition comprising the steps of bringing an emulsifying dispersant which contains polysaccharides with particulate structures as a main component thereof into contact with components to be emulsified and/or components to be dispersed, mixing them to emulsify and/or disperse them, and blending other components for the cosmetic composition therein.

13. A method for producing a cosmetic composition as claimed in claim 12, wherein said emulsifying dispersant Which includes polysaccharides with particulate structures as a main component is mixed with said components to be emulsified and/or said components to be dispersed in the weight ratio of from 1 : 50 to 1 : 1000.

14. A method for producing a cosmetic composition as claimed in one of claims 12 and 13, wherein said polysaccharides with particulate structures contain at least one of fucose, glucose, glucuronic acid and rhamnose as a constituting monosaccharide.

15. A producing method of a cosmetic composition, as claimed in one of claims 12 through 14, wherein said polysaccharides with particulate structures contain at least the polysaccharide represented by the following formula (1).

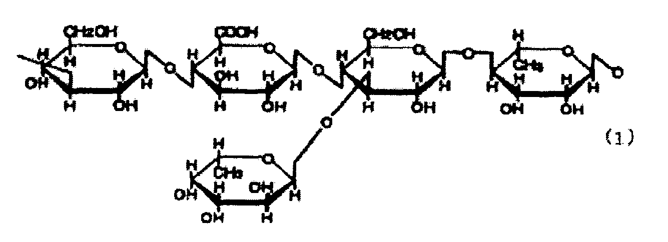

16. A producing method of a cosmetic composition, as claimed in one of claims 14 and 15, wherein said emulsifying dispersant which includes said polysaccharides with particulate structures is produced by adding urea to an aqueous solution containing said polysaccharide represented by the formula (1).
